(19) Europäisches Patentamt
European Patent Office
Office européen des brevets

(11) Publication number: **0 030 433**
**B1**

(12) **EUROPEAN PATENT SPECIFICATION**

(45) Date of publication of patent specification: **27.06.84**

(51) Int. Cl.³: **A 01 N 47/36,**
C 07 D 239/48,
C 07 D 251/18

(21) Application number: **80304288.6**

(22) Date of filing: **28.11.80**

(54) **Novel sulfonamides, compositions containing them, and methods for controlling the growth of undesired vegetation using them.**

(30) Priority: **30.11.79 US 98780**
**22.10.80 US 196266**

(43) Date of publication of application:
**17.06.81 Bulletin 81/24**

(45) Publication of the grant of the patent:
**27.06.84 Bulletin 84/26**

(84) Designated Contracting States:
**AT BE CH DE FR GB IT LI LU NL SE**

(56) References cited:
EP - A - 0 001 485
EP - A - 0 001 514
EP - A - 0 001 515
EP - A - 0 005 986
EP - A - 0 007 687
EP - A - 0 009 419
EP - A - 0 010 560
EP - A - 0 013 480
US - A - 4 127 405
US - A - 4 169 719

(73) Proprietor: **E.I. DU PONT DE NEMOURS AND COMPANY**
**Legal Department 1007 Market Street**
**Wilmington Delaware 19898 (US)**

(72) Inventor: **O'Grady, William Robert**
**13 Chancellor Drive**
**Newark Delaware 19713 (US)**

(74) Representative: **Hildyard, Edward Martin et al,**
**Frank B. Dehn & Co. European Patent Attorneys**
**Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 030 433 B1

Courier Press, Leamington Spa, England.

## Description

This invention relates to novel sulfonamides exhibiting herbicidal activity.

The presence of undesired vegetation causes substantial damage to useful crops, especially agricultural products that satisfy man's basic food and fiber needs, such as cotton, rice, corn, wheat, soybeans and the like. The current population explosion and concomitant world food and fiber shortage demand improvements in the efficiency of producing these crops. Preventing or minimising the loss of a portion of such valuable crops by killing or inhibiting the growth of undesired vegetation is one way of improving this efficiency.

A wide variety of materials useful for killing or inhibiting (controlling) the growth of undesired vegetation is available; such materials are commonly referred to as herbicides.

Netherlands Patent 121,788 claims herbicides such as:

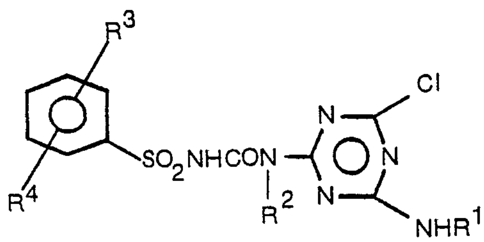

wherein

$R^1$ and $R^2 = C_1$—$C_4$ alkyl; and

$R^3$ and $R^4 = H$, Cl or $C_1$—$C_4$ alkyl.

In addition, U.S. Patent 4,127,405 teaches herbicidal compounds such as that of the formula

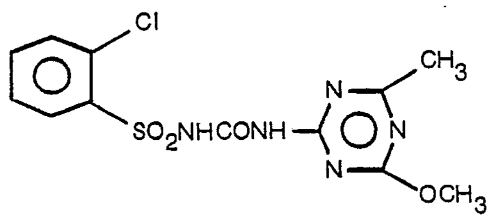

The need exists, however, for still more effective herbicides that destroy or control weeds without causing significant damage to useful crops.

We have now found novel compounds which may be useful as general or selective pre-emergence and post-emergence herbicides and/or plant growth regulants.

According to one aspect of the present invention there are provided compounds of general formula

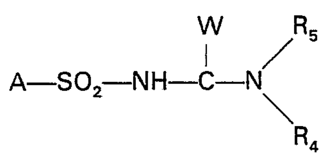 I

wherein

A is

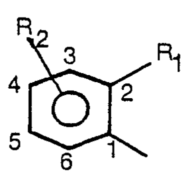

or

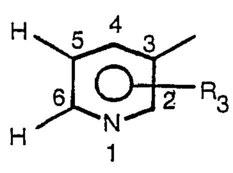

in which $R_1$ is $-\overset{O}{\overset{\|}{C}}-QR_6$, F, Cl, Br, $CH_3$, $OCH_3$, $NO_2$, $CF_3$, $SO_2NR_7R_8$, $S(O)_nR_8'$, $R_9\overset{O}{\overset{\|}{C}}$, $OSO_2R_{10}$ or $\underset{OCH_3}{SO_2N(CH_3)}$

in which Q is O, S or $\underset{R_{13}}{-N-}$ in which $R_{13}$ is H, $C_1$—$C_2$ alkyl or $OCH_3$,

$R_6$ is $C_1$—$C_4$ alkyl, $C_3$—$C_4$ alkenyl or $CH_3OCH_2CH_2$ or, when Q is O, then $R_6$ may also be $ClCH_2CH_2$, or $R_6$ and $R_{13}$ taken together form $—CH_2CH_2CH_2CH_2—$ or $—CH_2CH_2OCH_2CH_2—$,
$R_7$ and $R_8$, which may be the same or different, are $C_1$—$C_4$ alkyl provided that the total number of carbon atoms in $R_7$ and $R_8$ is less than or equal to five,
n is 0, 1 or 2,
$R_8'$ is $C_1$—$C_3$ alkyl,
$R_9$ is H or $C_1$—$C_4$ alkyl, and
$R_{10}$ is $C_1$—$C_4$ alkyl or $CF_3$,
$R_2$ is H, Cl, $CH_3$, $CF_3$, $NO_2$ or $OCH_3$, and
$R_3$ is H, F, Cl, Br, $CH_3$, $OCH_3$, $NO_2$, $CO_2R_{11}$ of $S(O)_nR_{12}$
in which $R_{11}$ is $C_1$—$C_4$ alkyl,
$R_{12}$ is $C_1$—$C_3$ alkyl, and
n is as defined above;
W is O or S;
$R_4$ is H or $CH_3$; and

$R_5$ is 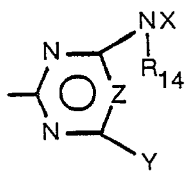 or 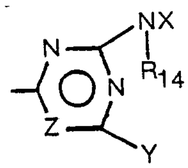

in which $R_{14}$ is H or $CH_3$,
Z is CH or N,
Y is F, Cl, Br, $C_1$—$C_2$ alkyl, $OCH_3$, $OCH_2CH_3$ or $CH_3OCH_2$, and

X is H, 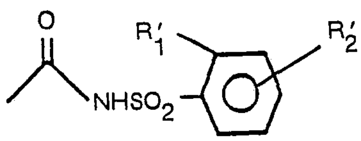 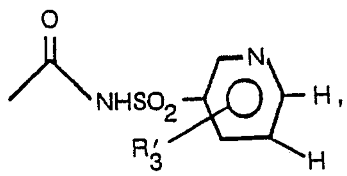, 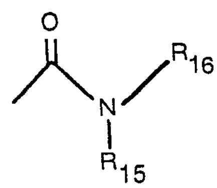,

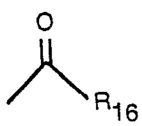, 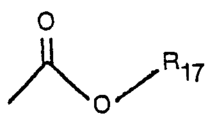, $CH_3SO_2$ or $CF_3SO$

in which $R_1'$ is $—\overset{O}{\overset{\|}{C}}—OR_6$, F, Cl, Br, $CH_3$, $OCH_3$, $NO_2$, $CF_3$, $SO_2NR_7R_8$, $S(O)_nR_8'$, $R_9\overset{O}{\overset{\|}{C}}$,

$OSO_2R_{10}$ or $\underset{\underset{OCH_3}{|}}{SO_2N(CH_3)}$

in which Q, $R_6$, $R_7$, $R_8$, $R_8'$, $R_9$, $R_{10}$ and n are as defined above,
$R_2'$ is H, Cl, $CH_3$, $CF_3$, $NO_2$ or $OCH_3$,
$R_3'$ is H, F, Cl, Br, $CH_3$, $OCH_3$, $NO_2$, $CO_2R_{11}$ or $S(O)_nR_{12}$
in which $R_{11}$, $R_{12}$ and n are as defined above,
$R_{15}$ is H, $C_1$—$C_4$ alkyl, $C_3$—$C_4$ alkenyl or $OCH_3$,
$R_{16}$ is H, $C_1$—$C_6$ alkyl or cycloalkyl, phenyl, or phenyl optionally substituted by $CF_3$, F, $NO_2$, CN, 1—2 Cl, 1—2 Br, 1—2 $CH_3$ or 1—2 $OCH_3$, and
$R_{17}$ is $C_1$—$C_6$ alkyl or cycloalkyl, phenyl, or phenyl optionally substituted by $CF_3$, F, $NO_2$, CN, 1—2 Cl, 1—2 Br, 1—2 $CH_3$ or 1—2 $OCH_3$;

with the provisos that:
1) when X is H, then Y is other than $CH_3$, $OCH_3$ or $OCH_2CH_3$;
2) when Y is F, Cl or Br, then $R_5$ is

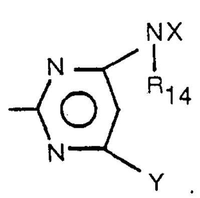 ;

3) when $R_{15}$ is $OCH_3$, then $R_{16}$ is $CH_3$;
4) when $R_{13}$ is $OCH_3$, then $R_6$ is $CH_3$; and
5) when A is

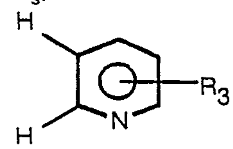

and $R_{14}$ is $CH_3$, then X is other than H; and the agriculturally suitable salts thereof.

## Preferred Compounds

*Preferred* for reasons of higher biological activity and/or lower cost and/or greater ease of synthesis are:

1) Compounds of the general formula I in which W is oxygen and $R_4$ is hydrogen;
2) Compounds of *Preferred 1)* in which $R_1$ and $R_1'$ are

$-\overset{\overset{O}{\|}}{C}-OR_6$, $NO_2$, $-Cl$, $SO_2NR_7R_8$, $SO_2\underset{\underset{CH_3}{|}}{N}(OCH_3)$, $S(O)_nR_8'$ and $OSO_2R_{10}$;

3) Compounds of *Preferred 2)* in which $R_5$ is

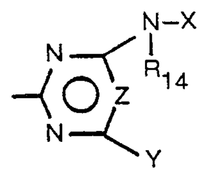

4) Compounds of *Preferred 3)* in which $R_1' = R_1$, $R_2' = R_2$ and $R_3' = R_3$ and $R_3$ is in 2-position of the pyridine ring;
5) Compounds of *Preferred 4)* in which $R_{15}$ is $C_1$—$C_2$ alkyl, $CH_2$—$CH=CH_2$, $OCH_3$ or H and $R_{16}$ is H, $C_1$—$C_3$ alkyl, phenyl or phenyl optionally substituted with $CF_3$, $NO_2$, 1—2 Cl, $CH_3$ or $OCH_3$;
6) Compounds of *Preferred 5)* in which $R_{17}$ is $C_1$—$C_3$ alkyl, phenyl or phenyl optionally substituted with Cl, Br, $CH_3$ or $OCH_3$;
7) Compounds of *Preferred 6)* in which $R_2$ is H;
8) Compounds of *Preferred 7)* in which $R_6$ is $C_1$—$C_3$ alkyl or $CH_2CH=CH_2$ and $R_{13}$ is $CH_3$; $R_1$ is $NO_2$, $SO_2N(R_7,R_8)_2$, $SO_2\underset{\underset{OCH_3}{|}}{N}(CH_3)$, $SO_2R_8'$, $OSO_2CH_3$ and $R_3$ is Cl, or $SO_2CH_3$; provided that the total number of carbons for $(R_7,R_8)$ is $\leq 4$;
9) Compounds of *Preferred 8)* in which Z is CH;
10) Compounds of *preferred 9)* in which X is

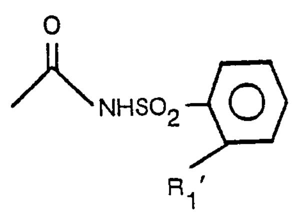

11) Compounds of *Preferred 9)* in which X is

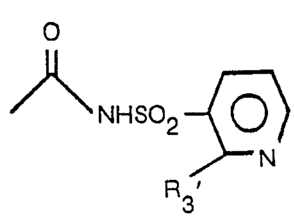

12) Compounds of *Preferred 9)* in which X is H;

13) Compounds of *Preferred 9)* in which X is 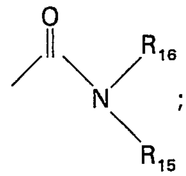 ;

14) Compounds of *Preferred 9)* in which X is 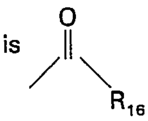

15) Compounds of *Preferred 9)* in which X is 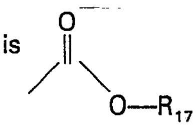

16) Compounds of *Preferred 9)* in which X is $CH_3SO_2$;

17) Compounds of *Preferred 9)* in which X is $CF_3SO_2$.

18) Compounds of *Preferred 13)* in which $R_{15}$ is H, $C_1$—$C_3$ alkyl or $OCH_3$ and $R_{16}$ is H or $C_1$—$C_3$ alkyl.

19) Compounds of *Preferred 14)* in which $R_{16}$ is $C_1$—$C_3$ alkyl.

20) Compounds of *Preferred 15)* in which $R_{17}$ is $C_1$—$C_3$ alkyl.

*Especially Preferred* for highest activity and/or lowest cost and/or greatest ease of synthesis are:
methyl 2-[[(4-amino-6-chloropyrimidin-2-yl)-aminocarbonyl]aminosulfonyl]benzoate;
dimethyl 2,2′-[6-chloropyrimidin-4,2-diyl-bis[(aminocarbonyl)aminosulfonyl]]-bis[benzoate];
N-[4-[[(2-chlorophenyl)sulfonylamino]carbonylamino]-6-methyl-1,3,5-triazin-2-yl]acetamide;
methyl 2-[[[4-(acetylamino)-6-methyl-1,3,5-triazin-2-yl]aminocarbonyl]aminosulfonyl]benzoate.

Synthesis

Synthetic methods for the preparation of compounds of formula I i.e. of formulae II, III, IV and V are shown in the following Equations 1A, B, C, and D.

Compounds of Formulae II—V are conveniently prepared by reacting either one or two equivalents of an appropriately substituted benzenesulfonylisocyanate or pyridine sulfonyl isocyanate with an appropriately substituted diaminopyrimidine or triazine.

EQUATION 1

A

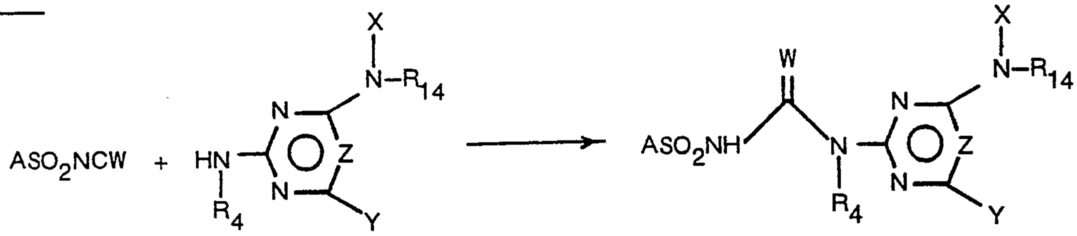

II

B

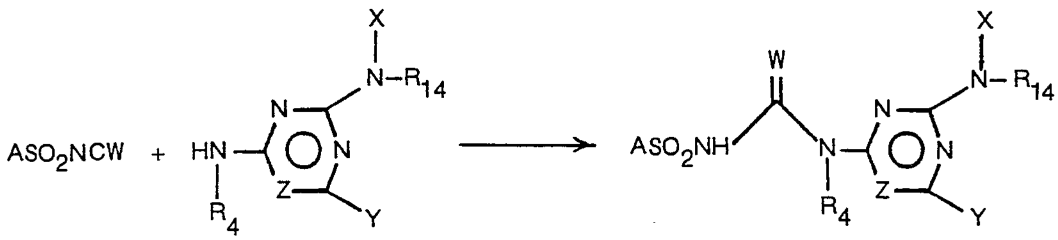

III

C

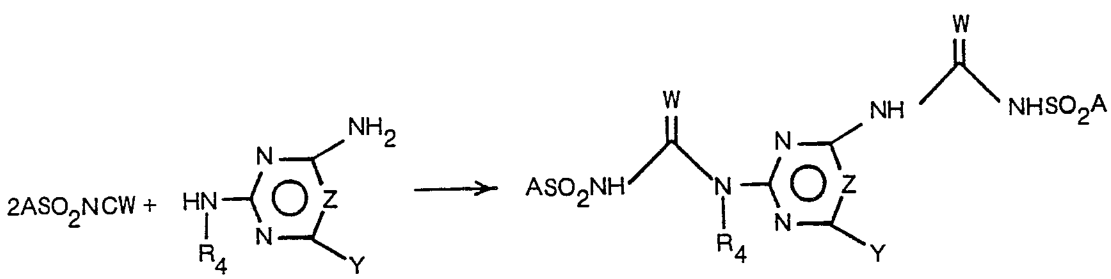

IV

D

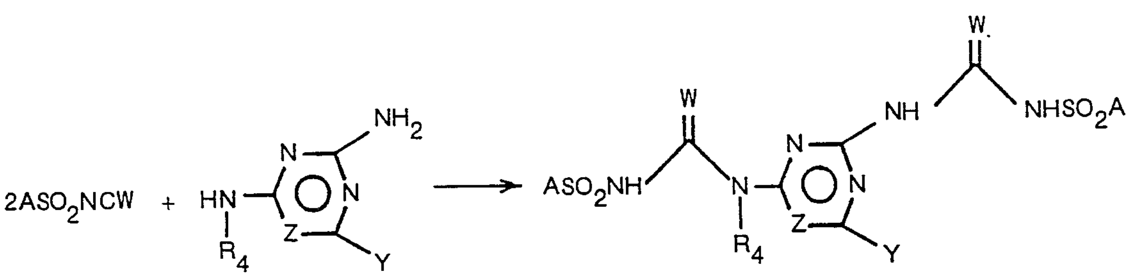

V

The reactions of Equations 1 are best carried out in inert aprotic organic solvents such as acetonitrile, tetrahydrofuran or methylene chloride. The reaction temperature can range from 25°C to 81°C. The reaction time can range from 3—96 hours depending upon the reactivity of the aminoheterocycle. In some cases, the desired product crystallizes from the reaction medium and may be filtered. Other products which are soluble in the reaction medium are isolated by evaporation of the solvent, trituration of the residue with solvents such as ethyl ether, 1-chlorobutane or hexanes and filtration.

Other compounds in the scope of this invention are prepared as shown in Equation 2.

EQUATION 2

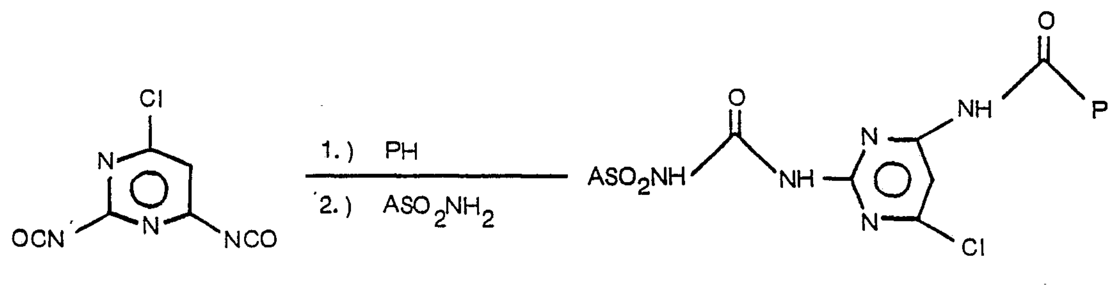


XIII VI

where P is 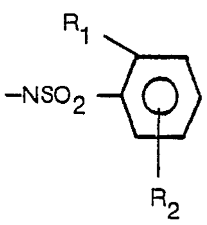 , 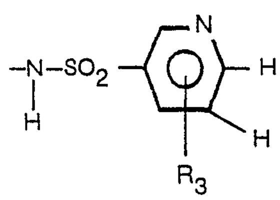 ,

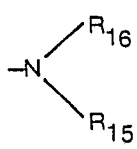 , or $-OR_{17}$ .

The intermediate 4-chloro-2,6-diisocyanatopyrimidine, XIII is prepared as described in Angew Chem. Int. Ed. Engl. *10,* 402 (1971). Compounds of Formula VI may be prepared by sequential addition of one equivalent of a nucleophile defined as PH, followed by one equivalent of the appropriately substituted benzenesulfonamide, or pyridine sulfonamide to XIII.

The reaction of Equation 2 is best carried out in inert aprotic organic solvents such as acetonitrile, tetrahydrofuran or methylene chloride.

Another route to compounds of this invention is shown in Equation 3A.

EQUATION 3

A

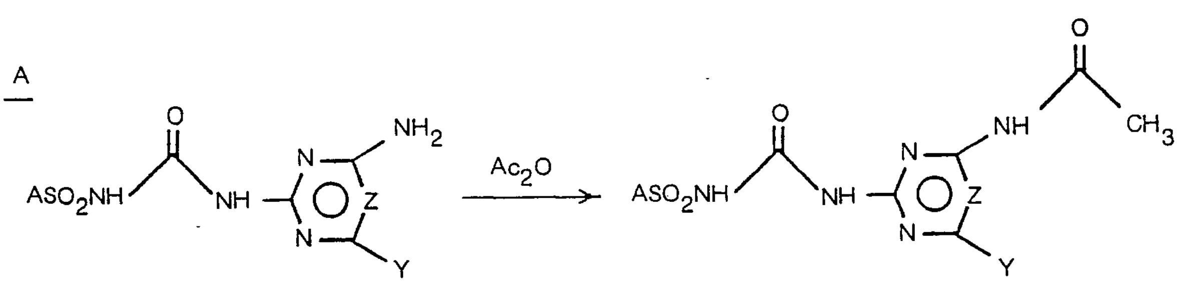


XIV VII

The synthetic method for the preparation of Formula VII is as described in Equation 3A. A mixture of the appropriately substituted XIV can be heated in acetic anhydride to give compounds of Formula VII.

Similarly, compounds of Formulae VIII, IX, X, XI and XII can be prepared according to Equations 3B, 3C, 3D, 3E and 3F.

EQUATIONS 3B, C, D, E and F

B

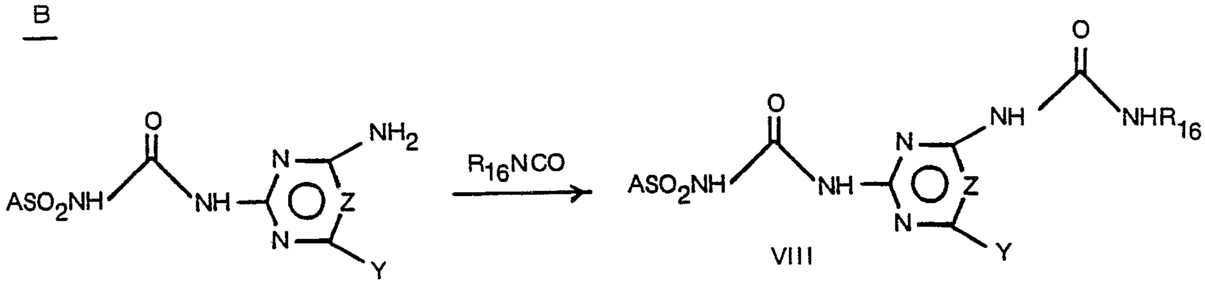


VIII

C

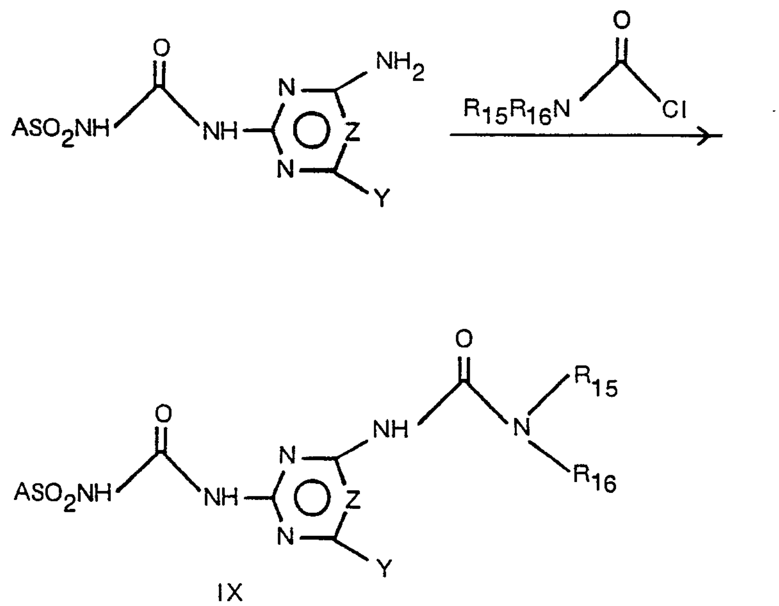

IX

D

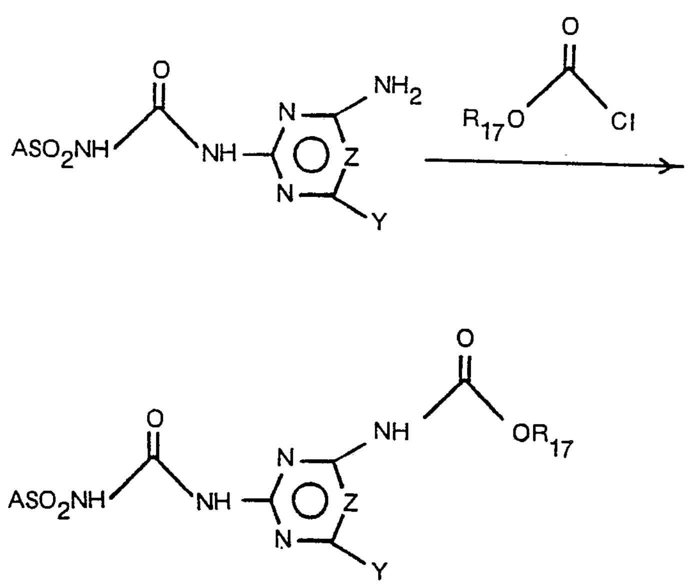

X

E

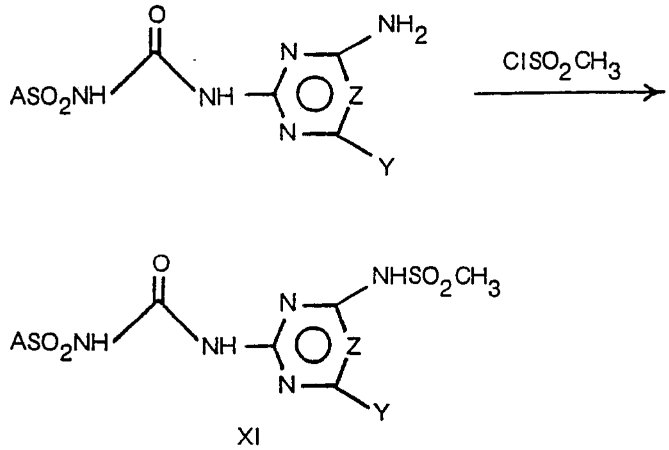


XI

F

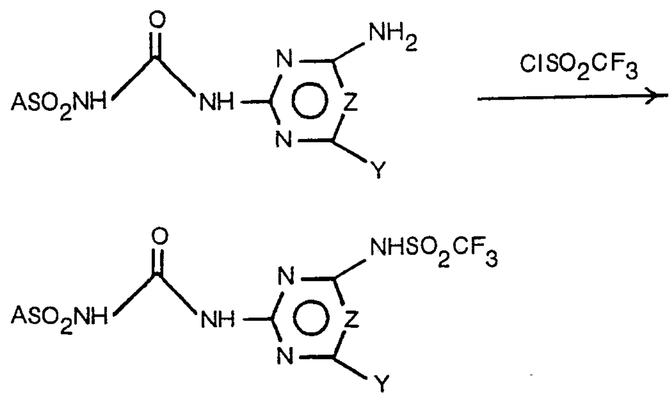


XII

Starting materials and intermediates for compounds not otherwise described herein are disclosed in U.S. patent 4,127,408 and "The Chemistry of Heterocyclic Compounds, The Pyrimidines", Interscience Publishers, D. J. Brown, S. F. Mason (1962).

The preparation of compounds of this invention are further illustrated by the following examples.

Example 1

*2-([(4-amino-6-chloropyrimidin-2-yl)aminocarbonyl]aminosulfonyl)benzoic acid, methyl ester*

To a warmed mixture containing 1.4 g (0.01 mole) 2,4-diamino-6-chloropyrimidine in 50 ml acetonitrile is added 2.4 g (0.01 mole) 2-carbomethoxybenzenesulfonylisocyanate. The reaction mixture was stirred at room temperature for four days. The reaction mixture was filtered and the precipitate washed with ether. The filtered solid was air dried and purified in the following manner. To an aqueous suspension of the reaction mixture was added *n*-propylamine and the aqueous mixture filtered. The resultant clear solution was carefully acidified with concentrated HCl to pH = 1—2. The mixture was filtered, washed with ether and air dried to yield a white solid, m.p. 170—172°C. The infrared spectrum showed characteristic absorption bands at 1740, 1700 $cm^{-1}$. The NMR and mass spectral data are consistent for the desired product.

Example 2

*2,2'[6-chloropyrimidin-2,4-diylbis(aminocarbonyl)aminosulfonyl]bis[benzoic acid], dimethyl ester*

To a warmed mixture containing 0.72 g (0.005 mole) 2,4-diamino-6-chloropyrimidine in 60 ml acetonitrile is added 4.3 g (0.018 mole) 2-carbomethoxybenzenesulfonylisocyanate. The reaction is heated to reflux temperature for four days. After cooling to room temperature, the resultant precipitate was filtered and dried to yield 0.8 g, m.p. 206—210°. The infrared spectrum shows characteristic absorption bands at 1725, 1675 $cm^{-1}$.

Elemental analysis:

Calc.: C, 42.14; H, 3.05; N, 13.40; Cl, 5.65; S, 10.23

Found: C, 42.2; H, 3.1; N, 14.2; Cl, 6.6; S, 10.3

Example 3

*N-[4-[2-Chlorophenyl-[sulfonylamino(carbonylamino)]]-6-methyl-1,3,5-triazin-2-yl]acetamide*

To 15 ml acetronitrile is added 1 g (0.006 mole) N-(4-amino-6-methyl-1, 3,5-triazin-2-yl)acetamide and a catalytic amount of dabco (triethylenediamine). To this suspension is added 1.8 g (0.008 mole) 2-chlorobenzenesulfonylisocyanate. The mixture is heated to 40°C and is kept at 40°C for 1 hour. The mixture is then cooled down to room temperature and is stirred at room temperature overnight. The solid is then collected by filtration, washed with acetonitrile, methylene chloride and n-butyl chloride, and dried to yield 2 g, m.p. 211—213°C. The infrared spectrum shows characteristic absorption bands at 1655 $cm^{-1}$, 1700 $cm^{-1}$. NMR is consistent for the desired product.

Compounds of Tables I—VI can be prepared according to Equations 1—3, as previously described.

TABLE I

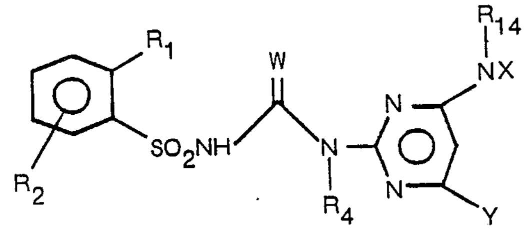

| $R_1$ | $R_2$ | $R_4$ | X | Y | W | $R_{14}$ |
|---|---|---|---|---|---|---|
| 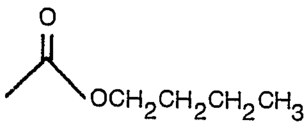 | H | H | H | Cl | O | H |
| 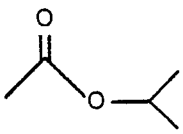 | 5-Cl | H | 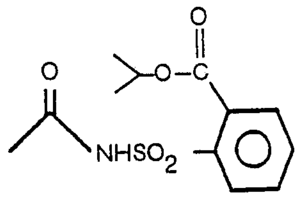 | Cl | O | H |
| Cl | H | H | 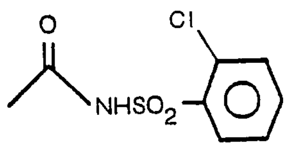 | Cl | S | H |
| $CH_3$ | 5-$CH_3$ | H | 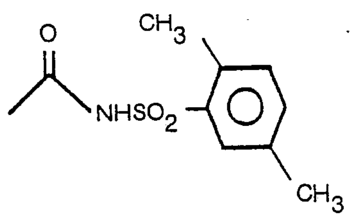 | Cl | O | H |
| 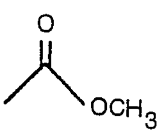 | H | H | 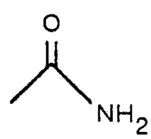 | Cl | O | H |
| 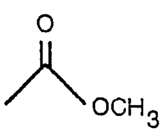 | H | H | 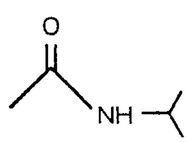 | $OCH_3$ | O | H |
| 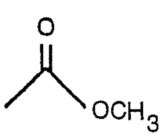 | H | H | 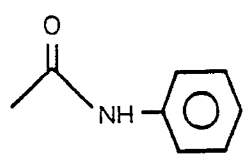 | $CH_3$ | O | H |
| 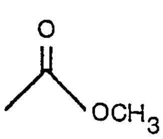 | H | H | 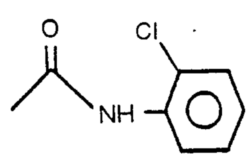 | Cl | O | H |

TABLE I (Continued)

| $R_1$ | $R_2$ | $R_4$ | X | Y | W | $R_{14}$ |
|---|---|---|---|---|---|---|
| 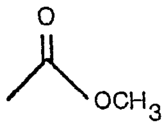 | H | H | 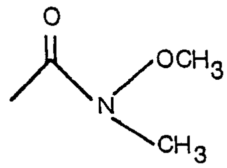 | Cl | O | H |
| 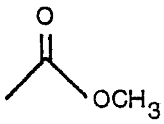 | H | H | 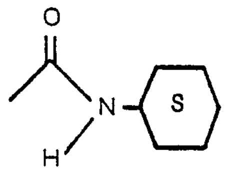 | Cl | O | H |
| Cl | 6-Cl | H | H | Cl | O | H |
| Cl | 6-Cl | H | 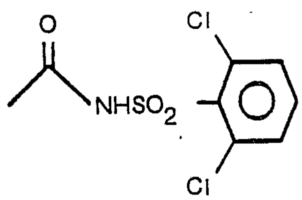 | Cl | O | H |
| Br | H | H | 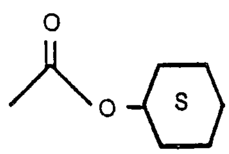 | F | O | H |
| $CH_3$ | 5-$CH_3$ | H | 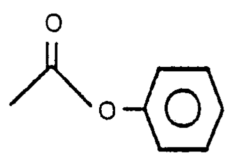 | Cl | O | H |
| $SCH_3$ | H | H | 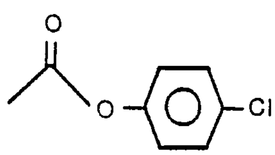 | Cl | O | H |
| $SO_2CH_3$ | H | H | 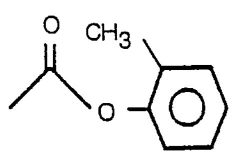 | Cl | O | H |
| $NO_2$ | H | H | 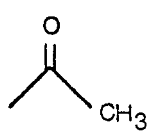 | Cl | O | H |
| $CF_3$ | H | H | 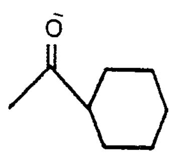 | Cl | O | H |

TABLE I (Continued)

| $R_1$ | $R_2$ | $R_4$ | X | Y | W | $R_{14}$ |
|---|---|---|---|---|---|---|
| $SO_2N(CH_3)_2$ | H | H | 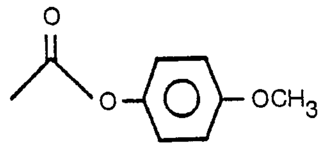 | Cl | O | H |
| 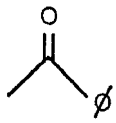 | H | H | 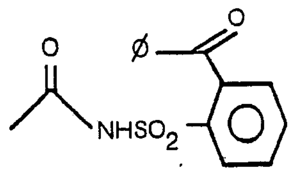 | Cl | O | H |
| 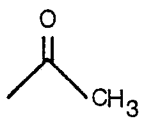 | H | H | 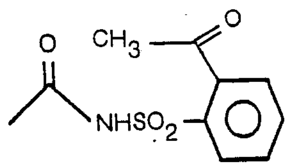 | Cl | O | H |
| 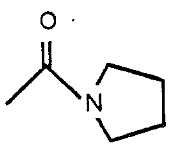 | H | H | 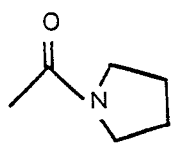 | Cl | O | H |
| $SCH_2CH_2CH_3$ | H | H | 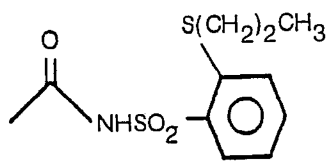 | $CH_3$ | O | H |
| $SO_2(CH_2)_2CH_3$ | H | H | 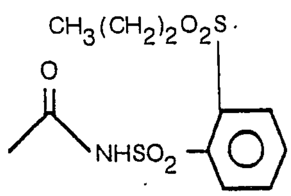 | $CH_3$ | O | H |
| 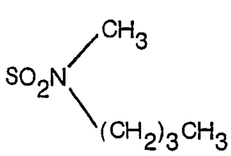 | H | H | 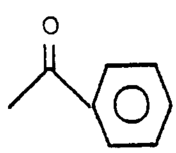 | $CH_3$ | O | H |
| 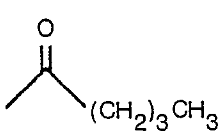 | H | H | 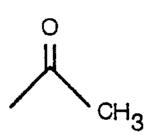 | Cl | O | H |
| $OSO_2CH_3$ | H | H | 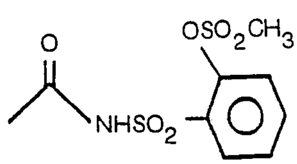 | Cl | O | H |

TABLE I (Continued)

| $R_1$ | $R_2$ | $R_4$ | X | Y | W | $R_{14}$ |
|---|---|---|---|---|---|---|
| $OSO_2(CH_2)_3CH_3$ | H | H | 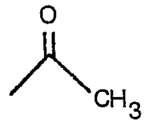 | Cl | O | H |
| $OSO_2CF_3$ | H | H | 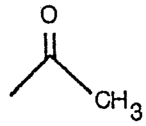 | Cl | O | H |
| 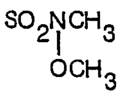 | H | H | 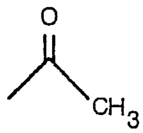 | Cl | O | H |
| Cl | 5-$CF_3$ | H | 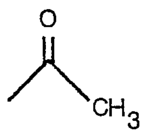 | Cl | O | H |
| Cl | 5-$NO_2$ | H | 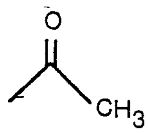 | Cl | O | H |
| Cl | 6-$OCH_3$ | H | 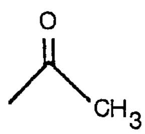 | Cl | O | H |
| Cl | H | $CH_3$ | 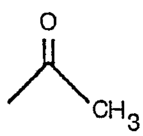 | Cl | O | H |
| 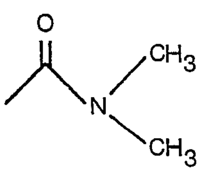 | H | H | 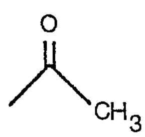 | Cl | O | H |
| 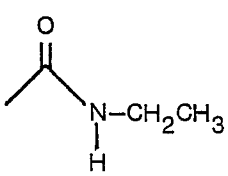 | H | H | 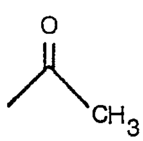 | Cl | O | H |
| 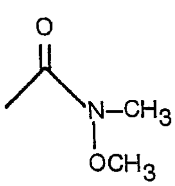 | H | H | 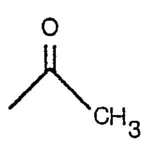 | Cl | O | H |

TABLE I (Continued)

| $R_1$ | $R_2$ | $R_3$ | X | Y | W | $R_{14}$ |
|---|---|---|---|---|---|---|
| $OCH_3$ | H | H | 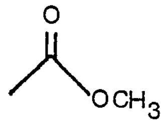 | $-CH_2CH_3$ | O | H |
| Cl | H | H | H | $-CH_2OCH_3$ | O | H |
| 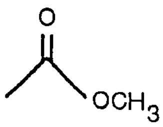 | H | H | 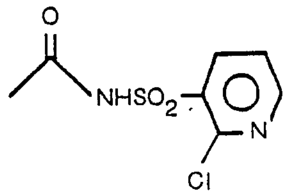 | Cl | O | H |
| 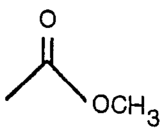 | H | H | 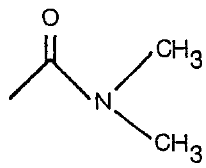 | Cl | O | H |
| 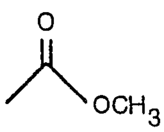 | H | H | 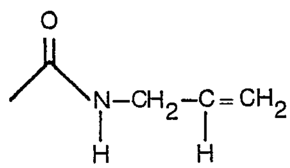 | Cl | O | H |
| 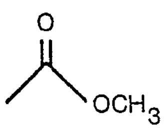 | H | H | 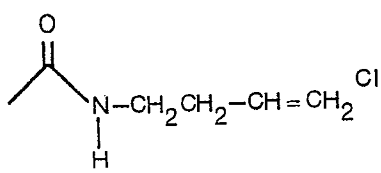 | Cl | O | H |
| Cl | H | H | 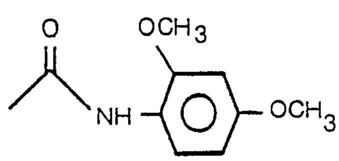 | Cl | O | H |
| Cl | H | H | 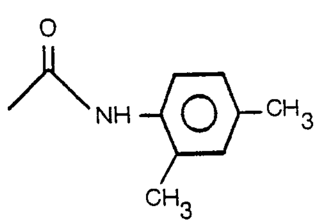 | Cl | O | H |
| Cl | H | H | 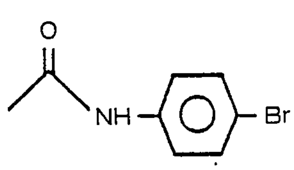 | Cl | O | H |

TABLE I (Continued)

| $R_1$ | $R_2$ | $R_4$ | X | Y | W | $R_{14}$ |
|---|---|---|---|---|---|---|
| Cl | H | H | 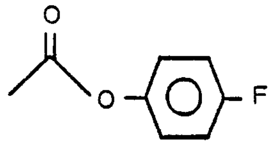 | Cl | O | H |
| Cl | H | H | 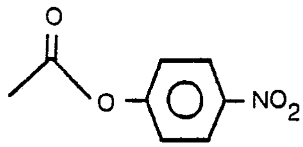 | Cl | O | H |
| Cl | H | H | 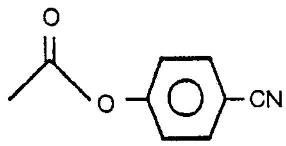 | Cl | O | H |
| Cl | H | H | 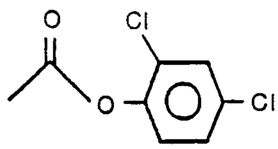 | Cl | O | H |
| Cl | H | H | 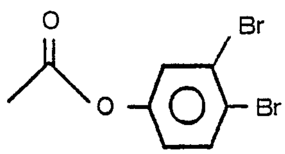 | Cl | O | H |
| Cl | H | H | 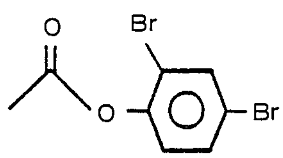 | Cl | O | H |
| Cl | H | H | 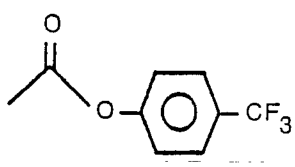 | Cl | O | H |
| Cl | H | H | H | Cl | O | H |
| Cl | H | H | H | Cl | O | H |
| Br | H | H | H | Cl | O | $CH_3$ |
| F | H | H | H | Cl | O | $CH_3$ |

TABLE II

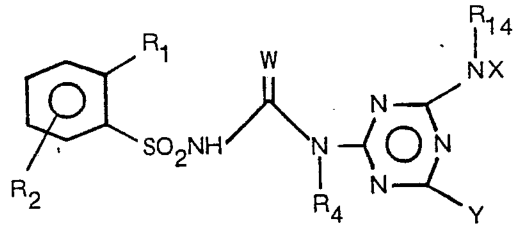

| $R_1$ | $R_2$ | $R_4$ | X | Y | W | $R_{14}$ |
|---|---|---|---|---|---|---|
| 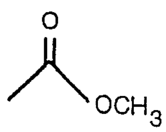 | H | H | 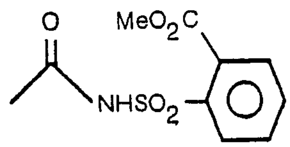 | $OCH_3$ | O | H |
| 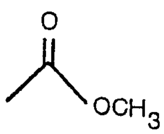 | H | H | 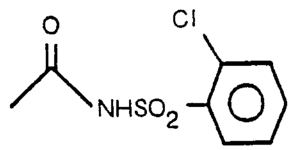 | $OCH_3$ | O | H |
| 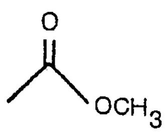 | H | H | 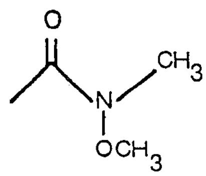 | $OCH_3$ | O | H |
| 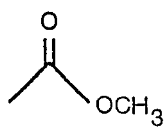 | H | H | 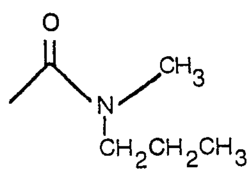 | $OCH_3$ | O | H |
| 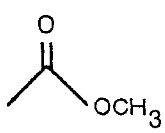 | H | H | 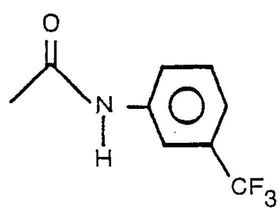 | $OCH_3$ | O | H |
| 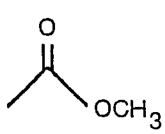 | H | H | 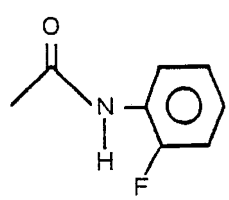 | $OCH_3$ | O | H |
| 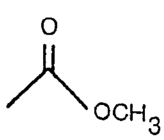 | H | H | 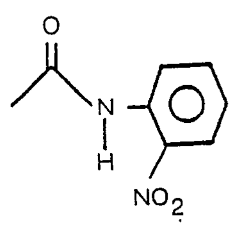 | $OCH_3$ | O | H |

TABLE II (Continued)

| $R_1$ | $R_2$ | $R_4$ | X | Y | W | $R_{14}$ |
|---|---|---|---|---|---|---|
| 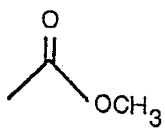 | H | H | 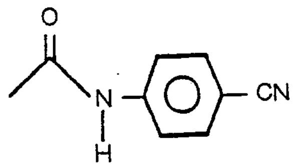 | $OCH_3$ | O | H |
| 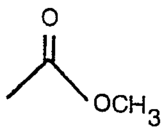 | H | H | 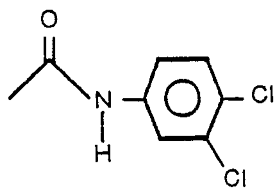 | $OCH_3$ | O | H |
| 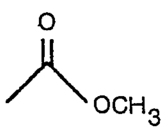 | H | H | 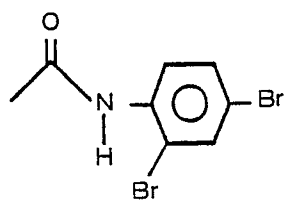 | $OCH_3$ | O | H |
| 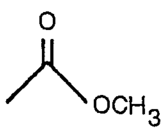 | H | H | 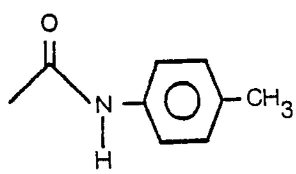 | $OCH_3$ | O | H |
| 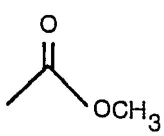 | H | H | 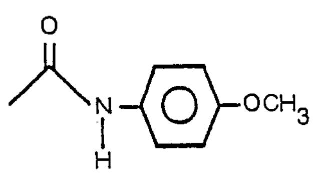 | $OCH_3$ | O | H |
| 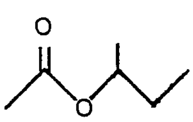 | H | H | 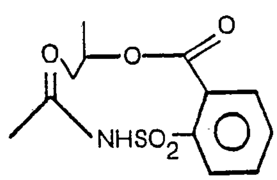 | $OCH_3$ | O | H |
| F | H | H | 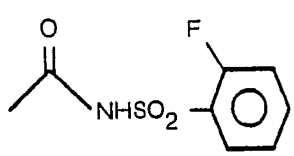 | $OCH_3$ | O | H |
| Cl | 6-Cl | H | 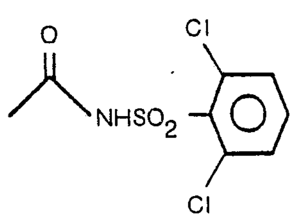 | $OCH_3$ | O | H |

TABLE II (Continued)

| $R_1$ | $R_2$ | $R_4$ | X | Y | W | $R_{14}$ |
|---|---|---|---|---|---|---|
| Br | H | H | 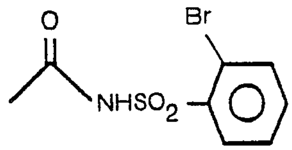 | $OCH_3$ | O | H |
| $CH_3$ | 5-$CH_3$ | H | 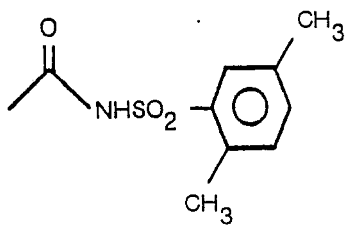 | $OCH_3$ | O | H |
| 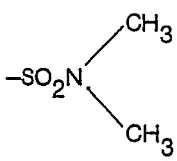 | H | H | 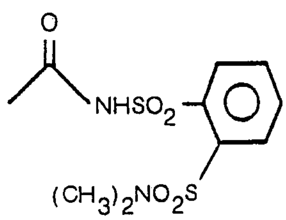 | $OCH_3$ | O | H |
| 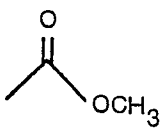 | H | H | 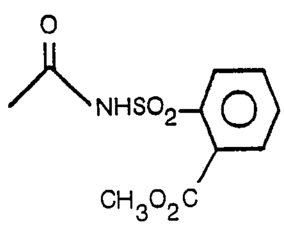 | $OCH_3$ | O | H |
| 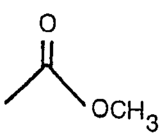 | H | H | 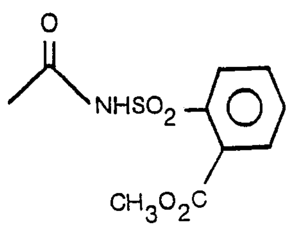 | $OCH_3$ | O | H |
| 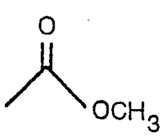 | H | H | 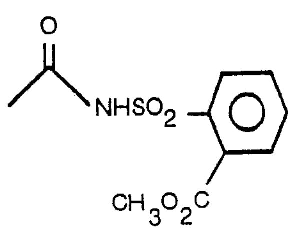 | $OCH_3$ | O | H |
| 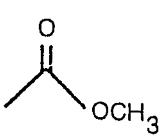 | H | H | 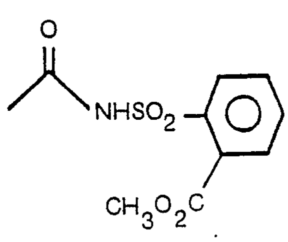 | $CH_3$ | O | H |

TABLE II (Continued)

| $R_1$ | $R_2$ | $R_4$ | X | Y | W | $R_{14}$ |
|---|---|---|---|---|---|---|
| 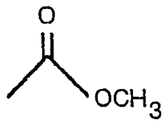 | H | H | 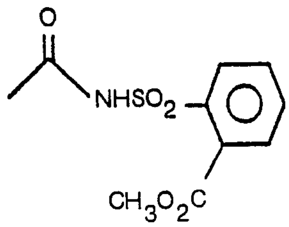 | $OCH_3$ | O | H |
| 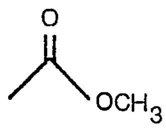 | H | H | 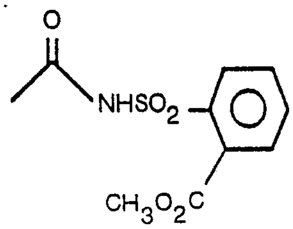 | $-CH_2CH_3$ | O | H |
| 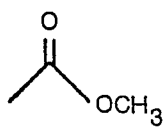 | H | H | 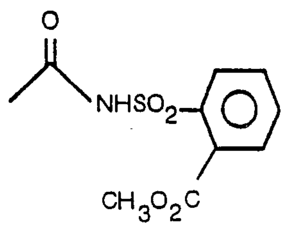 | $-OCH_2CH_3$ | O | H |
| $OCH_3$ | H | H | 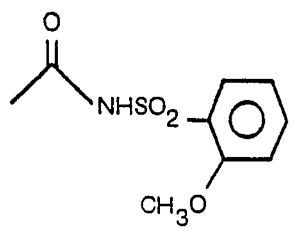 | $OCH_3$ | O | H |
| $SCH_3$ | H | H | 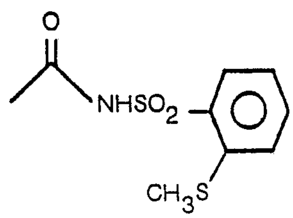 | $OCH_3$ | O | H |
| $SO_2CH_3$ | H | H | 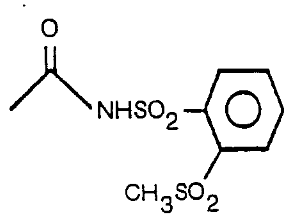 | $OCH_3$ | O | H |
| $NO_2$ | H | H | 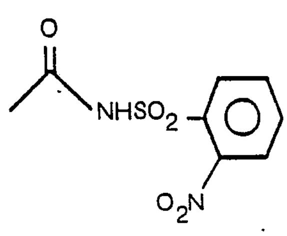 | $OCH_3$ | O | H |

TABLE II (Continued)

| $R_1$ | $R_2$ | $R_4$ | X | Y | W | $R_{14}$ |
|---|---|---|---|---|---|---|
| $CF_3$ | H | H | 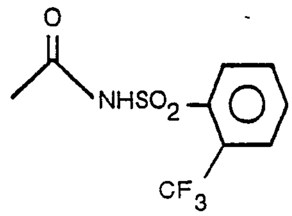 | $OCH_3$ | O | H |
| 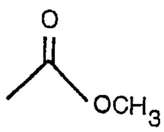 | H | H | H | | S | H |
| Cl | H | H | 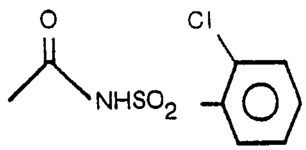 | $OCH_3$ | O | H |
| Cl | H | H | 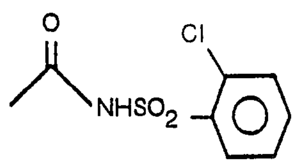 | $OCH_2CH_3$ | O | H |
| Cl | H | H | 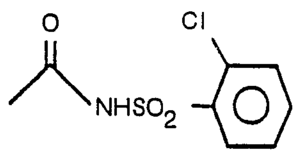 | $CH_3$ | O | H |

TABLE III

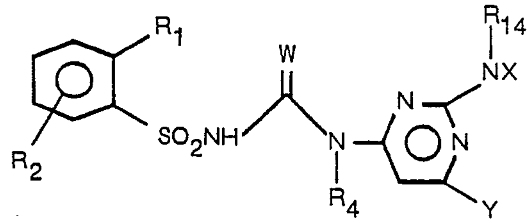

| $R_1$ | $R_2$ | $R_4$ | X | Y | W | $R_{14}$ |
|---|---|---|---|---|---|---|
| 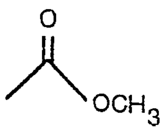 | H | H | H | Cl | O | H |
| Cl | H | H | H | Cl | O | H |
| 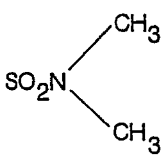 | H | H | H | Cl | O | H |
| 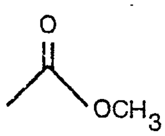 | H | H | 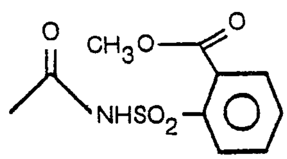 | $CH_3$ | O | H |
| 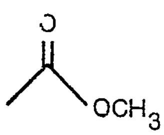 | H | H | 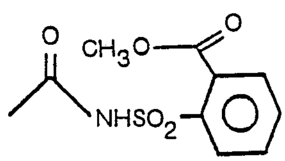 | Cl | O | H |
| Cl | H | H | 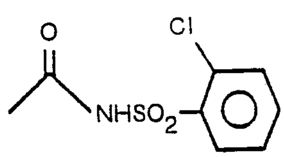 | Cl | O | H |

TABLE IV

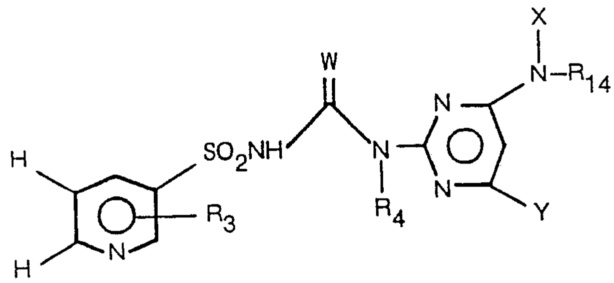

| $R_3$ | $R_4$ | X | Y | W | $R_{14}$ |
|---|---|---|---|---|---|
| 2-Cl | H | 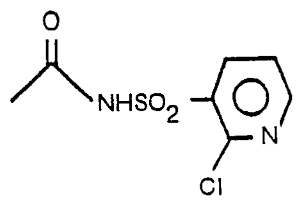 | Cl | O | H |
| 2-Cl | H | 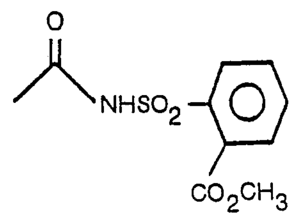 | Cl | O | H |
| 2-Cl | H | 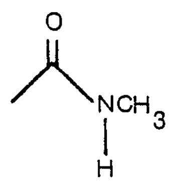 | Cl | O | H |
| 2-Cl | H | 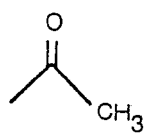 | Cl | O | H |
| 2-Cl | H | 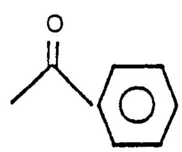 | Cl | O | H |
| 2-Cl | H | 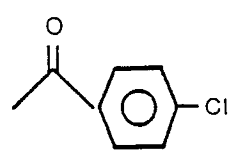 | Cl | O | H |
| 2-Cl | H | 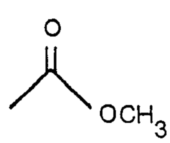 | Cl | O | H |

TABLE IV (Continued)

| $R_3$ | $R_4$ | X | Y | W | $R_{14}$ |
|---|---|---|---|---|---|
| 2-Cl | H | 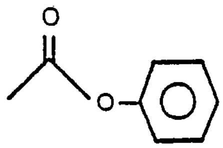 | Cl | O | H |
| 2-Cl | H | 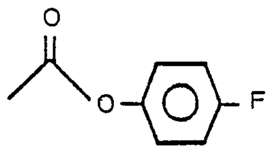 | Cl | O | H |
| 2-Cl | H | 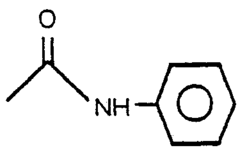 | Cl | O | H |
| 2-Cl | $CH_3$ | 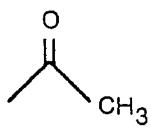 | Cl | O | H |
| 2-Cl | H | 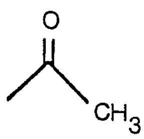 | Cl | O | $CH_3$ |
| 2-Br | H | 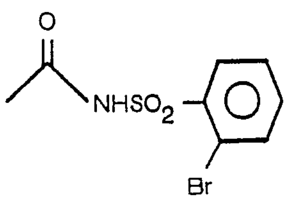 | Cl | O | H |
| 2-F | H | 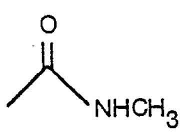 | Cl | O | H |
| 4-Cl | $CH_3$ | 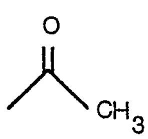 | Cl | O | H |
| 2-$CH_3$ | H | 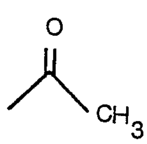 | Cl | O | H |

TABLE IV (Continued)

| $R_3$ | $R_4$ | X | Y | W | $R_{14}$ |
|---|---|---|---|---|---|
| 2-$OCH_3$ | H | 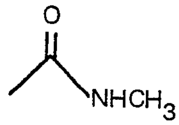 | Cl | O | H |
| 2-$NO_2$ | H | 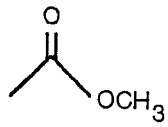 | Cl | O | H |
| 2 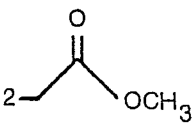 | H | 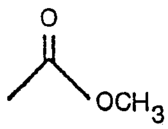 | Cl | O | H |
| 2 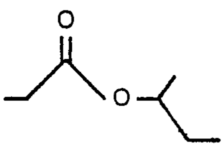 | H | 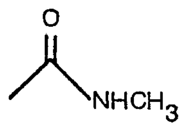 | Cl | O | H |
| 2-$SCH_3$ | H | 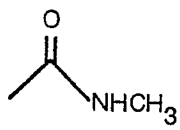 | Cl | O | H |
| 2-$SO_2CH_3$ | H | 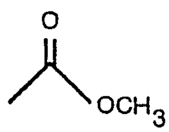 | Cl | O | H |
| 2-$SCH_2CH_2CH_3$ | H | 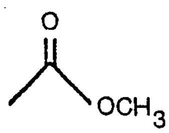 | Cl | O | H |
| H | H | 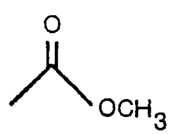 | Cl | O | H |

TABLE V

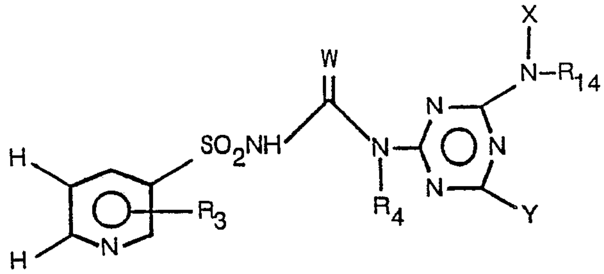

| $R_3$ | $R_4$ | X | Y | W | $R_{14}$ |
|---|---|---|---|---|---|
| 2-Cl | H | 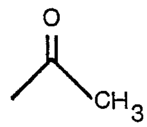 | $CH_3$ | O | H |
| 2-Cl | H | 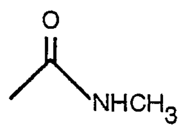 | $CH_3$ | O | H |
| 2-Cl | H | 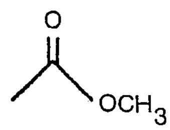 | $CH_3$ | O | H |
| 2-Br | H | 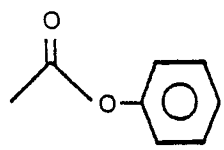 | $CH_3$ | O | H |
| 2-F | H | 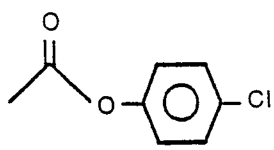 | $OCH_3$ | O | H |
| 2-Cl | H | 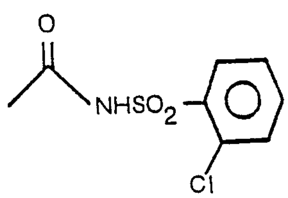 | $OCH_3$ | O | H |
| 2-Cl | H | 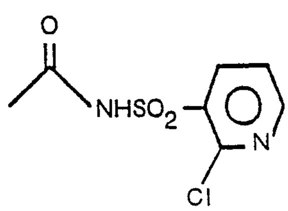 | $OCH_3$ | O | H |

TABLE VI

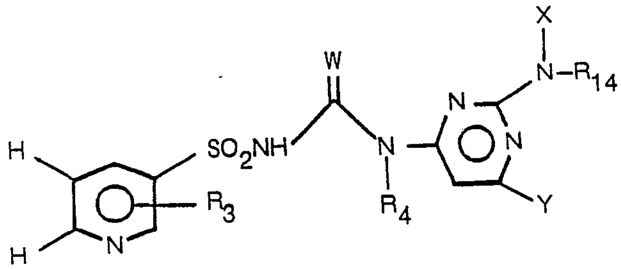

| $R_3$ | $R_4$ | X | W | $R_{14}$ | Y |
|---|---|---|---|---|---|
| 2-Cl | H | 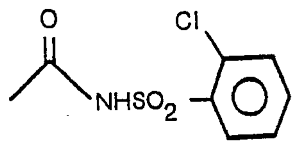 | O | H | Cl |
| 2-Cl | H | 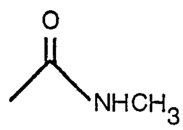 | O | H | Cl |
| 2-Cl | H | 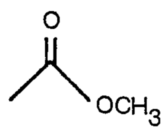 | O | H | Cl |
| 2-Cl | H | 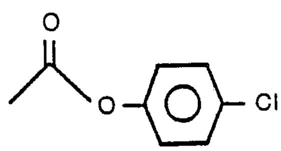 | O | H | Cl |
| 2-Cl | H | 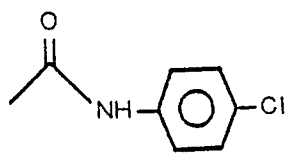 | O | H | Cl |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of a) about 0.1% to 20% surfactant(s) and b) about 1% to 99.9% solid or liquid diluent(s). More specifically, they may contain these ingredients in the following approximate proportions:

TABLE V

| | Weight % | | |
|---|---|---|---|
| | Active Ingredient | Diluent(s) | Surfactant(s) |
| Wettable Powders | 20—90 | 0—74 | 1—10 |
| Oil Suspensions, Emulsions, Solutions (including Emulsifiable Concentrates) | 3—50 | 40—95 | 0—15 |
| Aqueous Suspensions | 10—50 | 40—84 | 1—20 |
| Dusts | 1—25 | 70—99 | 0—5 |
| Granules and Pellets | 0.1—95 | 5—99.9 | 0—15 |
| High Stretch Compositions | 90—99 | 0—10 | 0—2 |

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "Mc Cutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foam, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering,* December 4, 1967, pp. 147ff. and "Perry's Chemical Engineer's Handbook", 4th Ed., McGraw-Hill, New York, 1963, pp. 8—59ff.

For further information regarding the art of formulation, see for example:

H. M. Loux, U.S. Patent 3,235,361, February 15, 1966, Col. 6, line 16 through Col. 7, line 19 and Examples 10 through 41.

R. W. Luckenbaugh, U.S. Patent 3,309,192, March 14, 1967, Col. 5, line 43 through Col. 7, line 62 and Examples 8, 12, 15, 39, 41, 52, 53, 132, 138—140, 162—164, 166, 167 and 169—182.

H. Gysin and E. Knusli, U.S. Patent 2,891,855, June 23, 1959, Col. 3, line 66 through Col. 5, line 17 and Examples 1—4.

G. C. Klingman, "Weed Control as a Science", John Wiley & Sons, Inc., New York, 1961, pp. 81—96.

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 4

Wettable Powder

| | |
|---|---|
| Dimethyl 2,2'-[6-chloropyrimidine-2,4-diylbis[(aminocarbonyl)-aminosulfonyl]]bis[benzoate] | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 5

Granule

| | |
|---|---|
| wettable powder of Example 4 | 5% |
| attapulgite granules | 95% |
| (U.S.S. 20—40 mesh; 0.84—0.42 mm) | |

A slurry of wettable powder containing ≈25% solids is sprayed on the surface of attapulgite granules in a double-cone blender. The granules are dried and packaged.

Example 6

Oil Suspension

| | |
|---|---|
| Dimethyl 2,2'-[6-chloropyrimidine-2,4-diylbis-[(aminocarbonyl)-aminosulfonyl]]bis[benzoate] | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting suspension may be applied directly, but preferably after being extended with oils or emulsified in water.

Example 7

Low Strength Granule

| | |
|---|---|
| Dimethyl 2,2'-[6-chloropyrimidine-2,4-diylbis[(aminocarbonyl)-aminosulfonyl]]bis[benzoate] | 0.1% |
| attapulgite granules | 99.9% |
| (U.S.S. 20—40 mesh) | |

The active ingredient is dissolved in a solvent and the solution is sprayed upon dedusted granules in a double cone blender. After spraying of the solution has been completed, the material is warmed to evaporate the solvent. The material is allowed to cool and then packaged.

Example 8

Wettable Powder

| | |
|---|---|
| Methyl 2-[[(4-amino-6-chloropyrimidin-2-yl)aminocarbonyl]-aminosulfonyl]benzoate | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until the solids are essentially under 50 microns, reblended and packaged.

Example 9

Extruded Pellet

| | |
|---|---|
| Methyl 2-[[(4-amino-6-chloropyrimidin-2-yl)aminocarbonyl]-aminosulfonyl]benzoate | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 10

Wettable Powder

| | |
|---|---|
| Methyl 2-[[4-amino-6-chloropyrimidin-2-yl)aminocarbonyl]-aminosulfonyl]benzoate | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer mill to produce particles essentially all below 100 microns, the material is reblended and sifted through a U.S.S. No. 50 sieve (0.3 mm opening) and packaged.

Example 11

High Strength Concentrate

| | |
|---|---|
| Methyl 2-[[(4-amino-6-chloropyrimidin-2-yl)aminocarbonyl]-aminosulfonyl]benzoate | 99% |
| silica aerogel | 0.5% |
| synthetic amorphous silica | 0.5% |

The ingredients are blended and ground in a hammer mill to produce a material essentially all passing a U.S.S. No. 50 screen (0.3 mm opening). The concentrate may be formulated further if necessary.

Example 12

Aqueous Suspension

| | |
|---|---|
| Methyl 2-[[(4-amino-6-chloropyrimidin-2-yl)aminocarbonyl]-aminosulfonyl]benzoate | 40% |
| Polyacrylic acid thickener | 0.3% |
| Dodecylphenol polyethylene glycol ether | 0.5% |
| Disodium phosphate | 1% |
| Monosodium phosphate | 0.5% |
| Polyvinyl alcohol | 1.0% |
| Water | 56.7% |

The ingredients are blended and ground together in a sand mill to produce particles essentially all under 5 microns in size.

Example 13

Granule

| | |
|---|---|
| Methyl 2-[[(4-amino-6-chloropyrimidin-2-yl)aminocarbonyl]-aminosulfonyl]benzoate | 80% |
| wetting agent | 1% |
| crude ligninsulfonate salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1910—149 microns), and packaged for use.

The compounds of Formula I are useful as herbicides. They may be applied either pre- or post-emergence for the control of undesired vegetation in noncrop areas or for selective weed control in certain crops, e.g., wheat. Some of these compounds are useful for the pre- and/or post-emergence control of nutsedge. By properly selecting rate and time of application, compounds of this invention may be used to modify plant growth beneficially.

The precise amount of the compound of Formula I to be used in any given situation will vary according to the particular end result desired, the use involved, the weeds to be controlled, the soil type, the formulation and mode of application, weather conditions, etc. Since so many variables play a role, it is not possible to state a rate of application suitable for all situations. Broadly speaking, the compounds of this invention are used at levels of about 0.01 to 20 kg/ha with a preferred range of 0.1 to 10 kg/ha. The lower rates of the range will generally be selected for lighter soils, for selective weed control in crops, or in situations where maximum persistence is not necessary. Some of the compounds of Formula I can be used at very low rates for plant growth modification, but higher rates may also be useful, depending on factors such as crop being treated, timing of treatment, etc.

The compounds of Formula I may be combined with other herbicides and are particularly useful in combination with 3-(3,4-dichlorophenyl)-1,1-dimethylurea, the triazines, such as 2-chloro-4-(ethylamino)-6-(isopropylamino)-*s*-triazine, the uracils such as 5-bromo-3-*sec*-butyl-6-methyl uracil, *N*-(phosphonomethyl)glycine, 3-cyclohexyl-1-methyl-6-dimethylamino-*s*-triazine-2,4(1*H*,3*H*)-dione, *N*,*N*-dimethyl-2,2-diphenylacetamide, 2,4-dichlorophenoxyacetic acid (and closely related compounds), 4-chloro-2-butynyl-3-chlorophenylcarbamate (Carbyne), diisopropylthiolcarbamic acid, ester with 2,3-dichloroallyl alcohol (Avadex), diisopropylthiolcarbamic acid, *S*-(2,3,3-trichlorallyl) ester (Avadex BW), ethyl-*N*-benzoyl-*N*-(3,4-dichlorophenyl)-2-aminopropionate (Suffix), 1,2 - dimethyl - 3,5 - diphenyl-

pyrazolium methyl-sulfate (Avenge), methyl 2-[4-(2,4-dichlorophenoxy)-phenoxy]-propanoate (Hoelon), 4 - amino - 6 - *tert* - butyl - 3 - (methylthio) - 1,2,4 - triazin - 5(4H) - one (Lexone), 3-(3,4-dichlorophenyl)-1-methoxy-1-methylurea (Lorox), 3-isopropyl-1H-2,1,3-benzothiodiazin-(4)-3*H*-one 2,2-dioxidine, $\alpha,\alpha,\alpha$-trifluoro - 2,6 - dinitro - *N,N* - dipropyl - *p* - toluidine, 1,1′ - dimethyl - 4,4′ - bipyridinium ion, monosodium methanearsonate, 2-chloro-2′,6′-diethyl-(methoxymethyl) acetanilide, and 1,1-dimethyl-3-($\alpha,\alpha,\alpha$-trifluoro-*m*-tolyl) urea (Cotoran).

The activity of compounds of formula I was investigated in a number of greenhouse tests. The tests are described and the data resulting from them are shown below.

Test Procedure A

Seeds of crabgrass (*Digitaria* spp.), barnyardgrass (*Echinochloa crusgalli*), wild oats (*Avena fatua*), *Cassia tora*, morningglory (*Ipomoea* spp.), cocklebur (*Xanthium* spp.), sorghum, corn, soybean, rice, wheat and nutsedge tubers were planted in a growth medium and treated pre-emergence with the chemicals dissolved in a non-phytotoxic solvent. At the same time, cotton having five leaves (including cotyledonary ones), bush beans with the second trifoliate leaf expanding, crabgrass with two leaves, barnyardgrass with two leaves, wild oats with one leaf, cassia with three leaves (including cotyledonary ones), morningglory with four leaves (including the cotyledonary ones), cocklebur with four leaves (including the cotyledonary ones), sorghum with three leaves, corn with three leaves, soybean with two cotyledonary leaves, rice with two leaves, wheat with two leaves, and nutsedge with three-five leaves were sprayed. Treated plants and controls were maintained in a greenhouse for sixteen days, then all species were compared to controls and visually rated for response to treatment. The ratings are based on a numerical scale extending from 0 = no injury, to 10 = complete kill. The accompanying descriptive symbols have the following meanings:

G = growth retardation;
C = chlorosis/necrosis;
D = defoliation;
E = emergence inhibition;
H = formative effects; and
U = unusual pigmentation.

The data for compounds tested by this procedure are in Table A.

TABLE A

| | 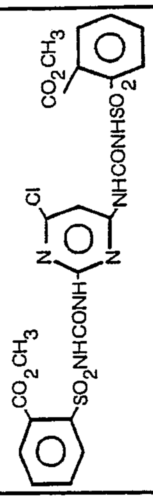 | 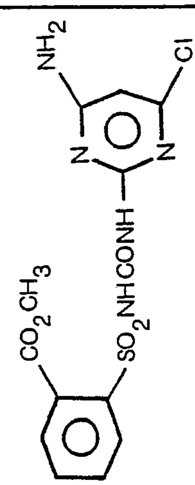 |
|---|---|---|
| Rate, kg/ha | 0.4 | 0.4 |
| POST-EMERGENCE | | |
| Bushbean | 8D, 7G, 6Y | 9C |
| Cotton | 3C, 3H, 6G | 5C, 9G |
| Morningglory | 4C, 9G | 10C |
| Cocklebur | 2C, 9G | 9C |
| Cassia | 3C, 8G | 6C, 9G |
| Nutsedge | 9G | 9C |
| Crabgrass | 2C, 7G | 2C, 9H |
| Barnyardgrass | 9C | 10C |
| Wild Oats | 2C, 9G | 3C, 9G |
| Wheat | 2C, 3G | 3C, 8G |
| Corn | 3C, 9H | 6U, 9G |
| Soybean | 2C, 3H, 9G | 4C, 9G |
| Rice | – | 5C, 9G |
| Sorghum | 4C, 9G | 10C |
| PRE-EMERGENCE | | |
| Morningglory | 8G | 9G |
| Cocklebur | 9G | 9H |
| Cassia | 7G | 5C, 9G |
| Nutsedge | 10E | 10E |
| Crabgrass | 1C, 3G | 6G |
| Barnyardgrass | 2C, 9H | 9H |
| Wild Oats | 1C, 8G | 3C, 9H |
| Wheat | 7G | 2C, 9H |
| Corn | 1C, 9G | 5C, 9G |
| Soybean | 6H | 9H |
| Rice | 10E | 10E |
| Sorghum | 6C, 9G | 3C, 9H |

Test Procedure B

Two plastic bulb pans were filled with fertilized and limed Fallsington silt loam soil. One pan was planted with corn, sorghum, Kentucky bluegrass and several grassy weeds. The other pan was planted with cotton, soybeans, purple nutsedge (*Cyperus rotundus*), and several broadleaf weeds. The following grassy and broadleaf weeds were planted: crabgrass (*Digitaria sanguinalis*), barnyardgrass (*Echinochloa crusgalli*), wild oats (*Avena fatua*), johnsongrass (*Sorghum halepense*), dallisgrass (*Paspalum dilatatum*), giant foxtail (*Setaria faberii*), cheatgrass (*Bromus secalinus*), mustard (*Brassica arvensis*), cocklebur (*Xanthium pensylvanicum*), pigweed (*Amaranthus retroflexus*), morningglory (*Ipomoea hederacea*), cassia (*Cassia tora*), teaweed (*Sida spinosa*), velvetleaf (*Abutilon theophrasti*), and jimsonweed (*Datura stramonium*). A 12.5 cm diameter plastic pot was also filled with prepared soil and planted with rice and wheat. Another 12.5 cm pot was planted with sugarbeets. The above four containers were treated preemergence with several test compounds within the scope of the invention.

Twenty-eight days after treatment, the plants were evaluated and visually rated for response to the chemical treatments utilizing the rating system described previously for Test A. The data are summarized in Table B.

TABLE B

PRE-EMERGENCE ON FALLSINGTON SILT LOAM

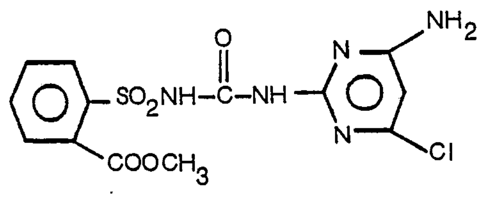

| Rate, kg/ha | 0.03 | 0.06 | 0.25 |
|---|---|---|---|
| | | | |
| Crabgrass | 5G | 7G | 8G, 3C |
| Barnyardgrass | 6G, 3H | 8G, 5H | 10C |
| Sorghum | 10C | 10C | 10C |
| Wild Oats | 3G, 2C | 6G, 3C | 10C |
| Johnsongrass | 9G, 6C | 9G, 9C | 10C |
| Dallisgrass | 5G | 6G | 9G, 9C |
| Giant Foxtail | – | 5G | 10C |
| Ky. bluegrass | 6G, 3C | 7G, 6C | 9C, 9C |
| Cheatgrass | 8G, 7C | 10C | 10C |
| Sugarbeets | 7G, 7C | 10C | 8G, 8C |
| Corn | 7G, 7H | 8G, 5C | 10C |
| Mustard | 10C | 10C | 10C |
| Cocklebur | – | 7G, 5H | 7G, 5H |
| Pigweed | – | – | – |
| Nutsedge | 10E | 10E | 10E |
| Cotton | 0 | 2H | 8G, 5H |
| Morningglory | 3G | 6G | 7G, 5H |
| Cassia | 5G | 8G, 8C | 8G, 9C |
| Teaweed | 0 | 8E | 10E |
| Velvetleaf | 7G, 5H | 10C | 10C |
| Jimsonweed | 0 | 0 | 5G, 6C |
| Soybean | 7G, 5H | 7G, 5H | 8G, 8H |
| Rice | 10E | 10E | 10E |
| Wheat | 0 | 4G | 8G, 8C |
| | | | |
| | | | |
| | | | |

TABLE B (Continued)

| PRE-EMERGENCE ON FALLSINGTON SILT LOAM | | |
|---|---|---|
| | 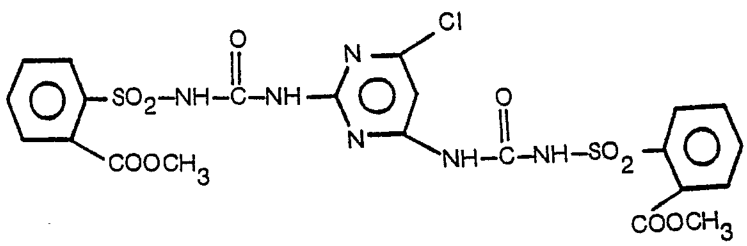 | |
| Rate, kg/ha | 0.03 | 0.12 |
| | | |
| Crabgrass | 0 | 0 |
| Barnyardgrass | 0 | 0 |
| Sorghum | 0 | 7G, 3H |
| Wild Oats | 0 | 0 |
| Johnsongrass | 0 | 7G, 3H |
| Dallisgrass | 0 | 0 |
| Giant Foxtail | 0 | – |
| Ky. bluegrass | 0 | 3G |
| Cheatgrass | 0 | 3G |
| Sugarbeets | 0 | 0 |
| Corn | 0 | 2G |
| Mustard | 0 | 8G, 3C |
| Cocklebur | 0 | 0 |
| Pigweed | – | – |
| Nutsedge | 2G | 8G |
| Cotton | 0 | 2G |
| Morningglory | 0 | 0 |
| Cassia | 0 | 0 |
| Teaweed | – | – |
| Velvetleaf | 0 | 4G, 3H |
| Jimsonweed | 0 | 0 |
| Soybean | 0 | 0 |
| Rice | 3G | 7G, 5H |
| Wheat | 0 | 0 |
| | | |
| | | |
| | | |

TABLE B (Continued)

PRE-EMERGENCE ON FALLSINGTON SILT LOAM

| | 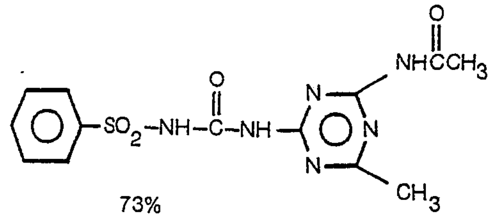 73% + 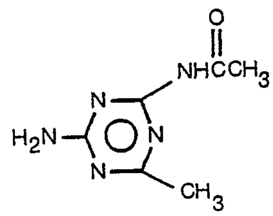 27% | | |
|---|---|---|---|
| Rate, kg/ha | 0.06 | 0.25 | |
| | | | |
| Crabgrass | 0 | 0 | |
| Barnyardgrass | 0 | 0 | |
| Sorghum | 3H | 5G, 3H | |
| Wild Oats | 0 | 5G | |
| Johnsongrass | 0 | 2G | |
| Dallisgrass | – | – | |
| Giant Foxtail | 0 | 0 | |
| Ky. bluegrass | 0 | 0 | |
| Cheatgrass | 0 | 4G | |
| Sugarbeets | 0 | 4G | |
| Corn | 0 | 2G | |
| Mustard | 8G, 8C | 8G, 9C | |
| Cocklebur | 0 | 0 | |
| Pigweed | 0 | 0 | |
| Nutsedge | 0 | 0 | |
| Cotton | 0 | 3G | |
| Morningglory | 3G, 2H | 7G, 5H | |
| Cassia | 0 | 0 | |
| Teaweed | 0 | 0 | |
| Velvetleaf | 2H | 4G, 3H | |
| Jimsonweed | 0 | 0 | |
| Soybean | 3H | 2G, 2H | |
| Rice | 4G | 7G, 8C | |
| Wheat | 0 | 6G, 5C | |
| | | | |
| | | | |
| | | | |

Test Procedure C

Two 25 cm (10 inch) in diameter plastic pans lined with polyethylene liners were filled with prepared Fallsington silt loam soil. One pan was planted with seeds of wheat (*Triticum aestivum*), barley (*Hordeum vulgare*), wild oats (*Avena fatua*), downy brome (*Bromus tectorum*), cheatgrass (*Bromus secalinus*), blackgrass (*Alopecurus myosuroides*), annual bluegrass (*Poa annua*), green foxtail (*Setaria viridis*), quackgrass (*Agropyron repens*), Italian ryegrass (*Lolium multiflorum*) and ripgut brome (*Bromus rigidus*). The other pan was planted with seeds of Russian thistle (*Salsola kali*), tansy mustard (*Descuraina pinnata*), smartweed (*Polygonum pensylvanicum*), tumble mustard (*Sisymbrium altissium*), kochia (*Kochia scoparia*), shepherd's purse (*Capsella bursa-pastoris*), *Matricaria inodora*, black night shade (*Solanum nigrum*), yellow rocket (*Barbarea vulgaris*), wild mustard (*Brassica kaber*) and wild buckwheat (*Polygonum convolvulus*). The above two pans were treated pre-emergence. At the same time two pans in which the above plant species were growing were treated post-emergence. Plant height at the time of treatment ranged from 1—15 cm depending on plant species.

The compounds applied were diluted with a non-phytotoxic solvent and sprayed over-the-top of the pans. An untreated control and a solvent alone control were included for comparison. All treatments were maintained in the greenhouse for 20 days at which time the treatments were compared to the controls and the effects visually rated. The recorded data are presented in Table C.

TABLE C

| | | |
|---|---|---|
| | 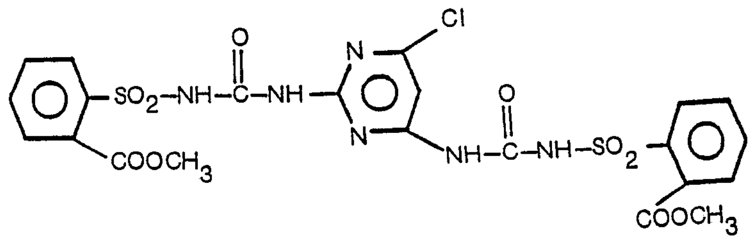 | |
| | Post-emergence | Pre-emergence |
| Rate, kg/ha | 0.06 | 0.06 |
| | | |
| Wheat | 0 | 0 |
| Barley | 0 | 0 |
| Wild oats | 0 | 1G |
| Downy brome | 2C, 3G | 1C, 1G |
| Cheatgrass | 3C, 6G | 6C, 7G |
| Blackgrass | 3C, 7G | 1C, 3G |
| Annual bluegrass | 5G | 5G |
| Green foxtail | 1C, 2G | 1G |
| Quackgrass | 2G | 1C, 2G |
| Italian ryegrass | 3G | 3C, 4G |
| Ripgut brome | 0 | 0 |
| Russian thistle | 2G | 2C, 3G |
| Tansy mustard | 3C, 6G | 8C, 8G |
| Smartweed | – | – |
| Jimhill mustard | 7C, 8G | 10C |
| Kochia | 2G | 3C, 4G |
| Shepherd's purse | 8C, 8G | 5C, 4G |
| False chamomile | 3C, 4G | 3C, 3G |
| Black nightshade | 3C, 2G | 0 |
| Yellow rocket | 7C, 8G | 6C, 5G |
| Wild mustard | 10C | 8C, 8G |
| Wild buckwheat | 3C, 3G | 3C, 4G |
| | | |
| | | |
| | | |
| | | |
| | | |

Test Procedure D

Purple nutsedge (*Cyperus rotundus*) tubers were planted about 2 cm deep in Fallsington silt loam soil contained in 10 cm diameter plastic pots. Five tubers were planted in each pot. Compounds of this invention were dissolved in an non-phytotoxic diluent and sprayed at 560 l/ha in four methods of application: soil surface, tuber/soil, soil incorporated, and post-emergence. The soil surface spray consisted of spraying the compound on the surface of the firmed covering soil. The tuber/soil spray consisted of spraying the compound on exposed tubers and subtending soil before adding the untreated covering soil. Soil incorporated treatment consisted in mixing the compound with the covering soil before using it to cover the tubers. The post-emergence treatment was sprayed on nutsedge foliage and the surrounding soil surface when nutsedge had emerged and grown to a height of about 12 cm. Pots receiving the post-emergence treatments were placed directly in the greenhouse. Pots receiving the other treatments were misted with about 0.3 cm water before being transferred to the greenhouse. Response ratings assessed after four weeks are recorded in Table D based on the same rating system as described in procedure A.

TABLE D

Nutsedge Control

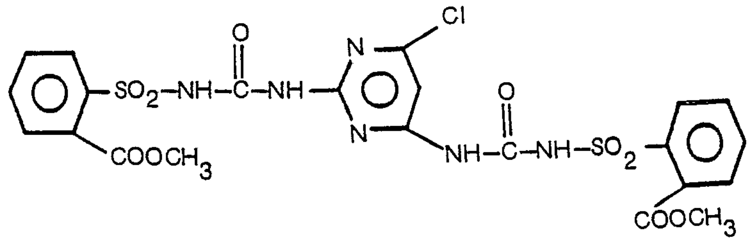

| Rate kg/ha | Response Rating (after 4 wks) | | | |
|---|---|---|---|---|
| | Preemerg. Soil Surface | Tuber Spray | Soil Incorp. | Postemerg. |
| 0.008 | 2G | 2G | 2G | 2G |
| 0.031 | 2G | 2G | 3G | 4G |
| 0.125 | 5E, 8G | 10E | 8E, 8G | 3C, 7G |

Test Procedure E

Twenty-five cm diameter plastic pots filled with Fallsington silt loam were planted to soybeans, cotton, alfalfa, corn, rice wheat, sorghum, velvetleaf (*Abutilon theophrasti*), sesbania (*Sesbania exaltata*), cassia (*Cassia tora*), morningglory (*Ipomoea hederacea*), jimsonweed (*Datura stramonium*), cocklebur (*Xanthium pensylvanicum*), crabgrass (*Digitaria spp.*), nutsedge (*Cyperus rotundus*), barnyardgrass (*Echinochloa crusgalli*), giant foxtail (*Setaria faberii*) and wild oats (*Avena fatua*). Approximately 2½ weeks after planting, the young plants and the soil around them were sprayed overall with the test chemicals dissolved in a nonphytotoxic solvent. Fourteen days after treatment, all species were compared to untreated controls and visually rated for response to treatment. The rating system was as described previously for Test A. The data presented in Table E.

TABLE E

| | 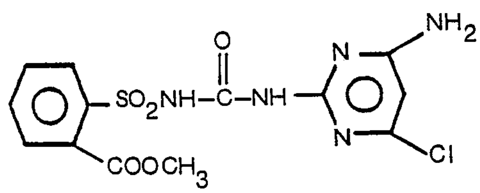 | | | |
|---|---|---|---|---|
| Rate, kg/ha | 0.25 | 0.06 | 0.015 | 0.007 |
| | | | | |
| Soybeans | 10C | 10C | 10C | 9G, 6C |
| Velvetleaf | – | – | – | 9G, 5C |
| Sesbania | 10C | 10C | – | 8G, 4C |
| Cassia | 10G, 9C | 10G, 7C | 10G, 7C | 6G, 2C |
| Cotton | 8G, 3C | 6G, 3C | 6G, 3C | 6G, 3C |
| Morningglory | 10C | 10G, 7C | 8G, 3C | 6G, 2C |
| Alfalfa | 10C | 10C | 9C | 7G, 2C |
| Jimsonweed | – | – | – | 0 |
| Cocklebur | 9C | – | 7G, 3C | 8G, 5C |
| Corn | 9G, 5G | 9G, 5G | 9G, 7H | 4G, 4H |
| Crabgrass | 9G, 4C | 9G | 1G | 0 |
| Rice | 9G, 4C | 9G, 3C | 7G, 3C | 7G, 2C |
| Nutsedge | 9G, 3C | 7G, 1C | 7G | 8G, 3C |
| Barnyardgrass | 10G, 6C | 9G, 5C | 9G, 5C | 7G, 1C |
| Wheat | 7G, 3C | 7G, 2C | 2G | 0 |
| Giant Foxtail | – | – | – | 8G, 3C |
| Wild Oats | 10G, 6C | 9G, 4C | 8G, 2C | 4G |
| Sorghum | 10G, 8C | 9G, 6C | 9G, 4C | 9G, 2C |
| | | | | |
| | | | | |
| | | | | |
| | | | | |
| | | | | |
| | | | | |
| | | | | |
| | | | | |
| | | | | |

**Claims for the Contracting States: BE CH DE FR GB IT LI LU NL SE**

1. Compounds of general formula

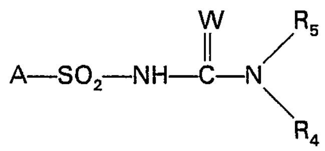

I

wherein
A is

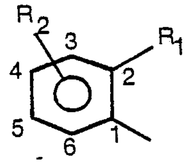

or

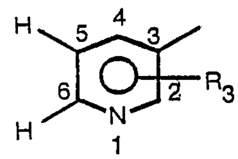

in which $R_1$ is $-\overset{O}{\overset{\|}{C}}-QR_6$, F, Cl, Br, $CH_3$, $OCH_3$, $NO_2$, $CF_3$, $SO_2NR_7R_8$, $S(O)_nR_8'$, $R_9\overset{O}{\overset{\|}{C}}$, $OSO_2R_{10}$ or $SO_2N(CH_3)(OCH_3)$

in which Q is O, S or $-N(R_{13})-$ in which $R_{13}$ is H, $C_1$—$C_2$ alkyl or $OCH_3$,

$R_6$ is $C_1$—$C_4$ alkyl, $C_3$—$C_4$ alkenyl or $CH_3OCH_2CH_2$, or, when Q is O, then $R_6$ may also be $ClCH_2CH_2$, or $R_6$ and $R_{13}$ taken together form $-CH_2CH_2CH_2CH_2-$ or $-CH_2CH_2OCH_2CH_2-$,
$R_7$ and $R_8$, which may be the same or different, are $C_1$—$C_4$ alkyl provided that the total number of carbon atoms in $R_7$ and $R_8$ is less than or equal to five,
n is 0, 1 or 2,
$R_8'$ is $C_1$—$C_3$ alkyl,
$R_9$ is H or $C_1$—$C_4$ alkyl, and
$R_{10}$ is $C_1$—$C_4$ alkyl or $CF_3$,
$R_2$ is H, Cl, $CH_3$, $CF_3$, $NO_2$ or $OCH_3$, and
$R_3$ is H, F, Cl, Br, $CH_3$, $OCH_3$, $NO_2$, $CO_2R_{11}$ or $S(O)_nR_{12}$
in which $R_{11}$ is $C_1$—$C_4$ alkyl,
$R_{12}$ is $C_1$—$C_3$ alkyl, and
n is as defined above;
W is O or S:
$R_4$ is H or $CH_3$ and

$R_5$ is

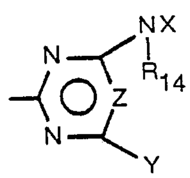

or

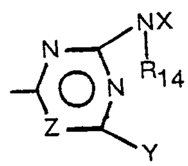

in which $R_{14}$ is H or $CH_3$,
Z is CH or N,
Y is F, Cl, Br, $C_1$—$C_2$ alkyl, $OCH_3$, $OCH_2CH_3$ or $CH_3OCH_2$, and

X is H,

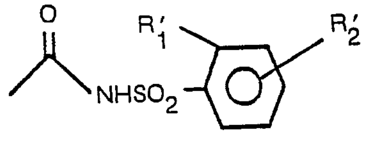

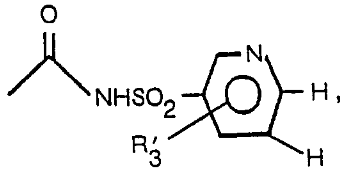

,

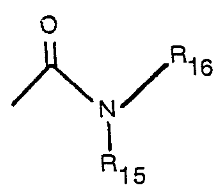

,

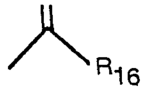

,

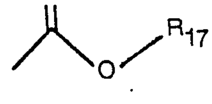

, $CH_3SO_2$ or $CF_3SO_2$,

in which $R_1'$ is $-\overset{\overset{O}{\|}}{C}-QR_6$, F, Cl, Br, $CH_3$, $OCH_3$, $NO_2$, $CF_3$, $SO_2NR_7R_8$, $S(O)_nR_8'$, $R_9\overset{\overset{O}{\|}}{C}$, $OSO_2R_{10}$ or $SO_2\underset{\underset{OCH_3}{|}}{N}(CH_3)$ in which Q, $R_6$, $R_7$, $R_8$, $R_8'$, $R_9$, $R_{10}$ and n are as defined above,

$R_2'$ is H, Cl, $CH_3$, $CF_3$, $NO_2$ or $OCH_3$
$R_3'$ is H, F, Cl, Br, $CH_3$, $OCH_3$, $NO_2$, $CO_2R_{11}$ or $S(O)_nR_{12}$ in which $R_{11}$, $R_{12}$ and n are as defined above,
$R_{15}$ is H, $C_1$—$C_4$ alkyl, $C_3$—$C_4$ alkenyl or $OCH_3$,
$R_{16}$ is H, $C_1$—$C_6$ alkyl or cycloalkyl, phenyl, or phenyl optionally substituted by $CF_3$, F, $NO_2$, CN, 1—2 Cl, 1—2 Br, 1—2, $CH_3$ or 1—2 $OCH_3$, and
$R_{17}$ is $C_1$—$C_6$ alkyl or cycloalkyl, phenyl, or phenyl optionally substituted by $CF_3$, F, $NO_2$, CN, 1—2 Cl, 1—2 Br, 1—2 $CH_3$ or 1—2 $OCH_3$;

with the provisos that:

1) when X is H, then Y is other than $CH_3$, $OCH_3$ or $OCH_2CH_3$;
2) when Y is F, Cl or Br, then $R_5$ is

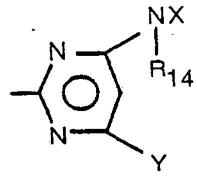

3) when $R_{15}$ is $OCH_3$, then $R_{16}$ is $CH_3$;
4) when $R_{13}$ is $OCH_3$, then $R_6$ is $CH_3$; and
5) when A is

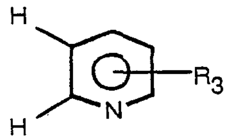

and $R_{14}$ is $CH_3$, then X is other than H;

and the agriculturally suitable salts thereof.

2. Compounds according to claim 1 in which W is O and $R_4$ is H.

3. Compounds according to claim 2 in which $R_1$ and/or $R_1'$ are

$-\overset{\overset{O}{\|}}{C}-QR_6$, $NO_2$, Cl, $SO_2NR_7R_8$, $SO_2\underset{\underset{CH_3}{|}}{N}(OCH_3)$, $S(O)_nR_8'$ or $OSO_2R_{10}$.

4. Compounds according to claim 3 in which $R_5$ is

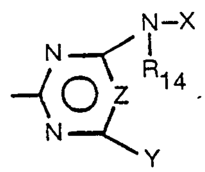

5. Compounds according to claim 4 in which $R_1'$ is the same as $R_1$, $R_2'$ is the same as $R_2$, $R_3'$ is the same as $R_3$, and $R_3$ is in the 2-position of the pyridine ring.

6. Compounds according to claim 5 in which $R_{15}$ is $C_1$—$C_2$ alkyl, $CH_2$—$CH{=}CH_2$, $OCH_3$ is H; and $R_{16}$ is H, $C_1$—$C_3$ alkyl, phenyl or phenyl optionally substituted by $CF_3$, $NO_2$, 1—2 Cl, $CH_3$ or $OCH_3$.

7. Compounds according to claim 6 in which $R_{17}$ is $C_1$—$C_3$ alkyl, phenyl or phenyl optionally substituted by Cl, Br, $CH_3$ or $OCH_3$.

8. Compounds according to claim 7 in which $R_2$ is H.

9. Compounds according to claim 8 in which $R_6$ is $C_1$—$C_3$ alkyl or $CH_2CH{=}CH_2$; $R_{13}$ is $CH_3$; $R_1$ is $NO_2$, $SO_2N(R_7,R_8)_2$, $SO_2\underset{\underset{OCH_3}{|}}{N}(CH_3)$,

$SO_2R_8'$ or $OSO_2CH_3$; and $R_3$ is Cl or $SO_2CH_3$; provided that the total number of carbon atoms in ($R_7$, $R_8$) is not greater than four.

10. Compounds according to claim 9 in which Z is CH.

11. Compounds according to claim 10 in which X is H,

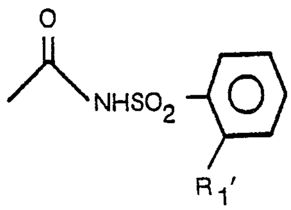
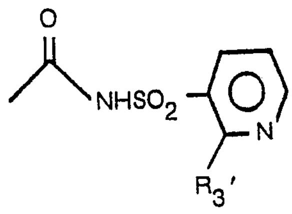
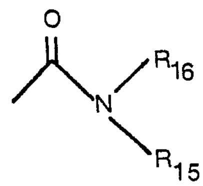

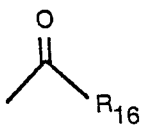
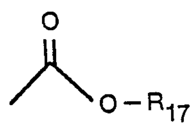
, $CH_2SO_2$ or $CF_3SO_2$.

12. Compounds according to claim 11 in which X is

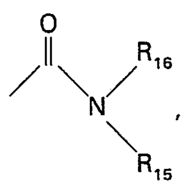
,

wherein $R_{15}$ is H, $C_1$—$C_3$ alkyl or $OCH_3$, and $R_{16}$ is H or $C_1$—$C_3$ alkyl.

13. Compounds according to claim 11 in which X is

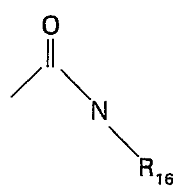

wherein $R_{16}$ is $C_1$—$C_3$ alkyl.

14. Compounds according to claim 11 in which X is

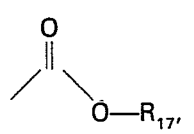

wherein $R_{17}$ is $C_1$—$C_3$ alkyl.

15. A compound according to claim 1 which is methyl 2-{[(4-amino-6-chloropyrimidin-2-yl)-aminocarbonyl]aminosulfonyl}-benzoate.

16. A compound according to claim 1 which is dimethyl 2,2′-{6-chloropyrimidin-4,2-diyl-bis[(aminocarbonyl)aminosulfonyl]}bis[benzoate].

17. A compound according to claim 1 which is N-{4-[((2-chlorophenyl)sulfonylamino)-carbonyl-amino]-6-methyl-1,3,5-triazin-2-yl}acetamide.

18. A compound according to claim 1 which is methyl 2-{[(4-(acetylamino)-6-methyl-1,3,5-triazin-2-yl)aminocarbonyl]aminosulfonyl}benzoate.

19. A composition suitable for controlling the growth of undesired vegetation which comprises an effective amount of a compound according to any one of claims 1 to 18 together with at least one surfactant, and/or solid or liquid diluent.

20. A method for controlling the growth of undesired vegetation in a locus which comprises applying to the locus an effective amount of a compound according to any one of claims 1 to 19.

**Claims for the Contracting State: AT**

1. A process for the preparation of compounds of general formula

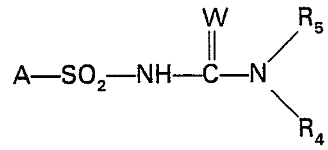

I

wherein

A is

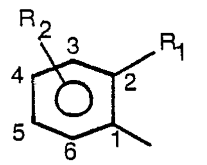

or

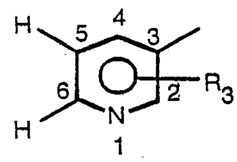

in which $R_1$ is $-\overset{O}{\overset{\|}{C}}-QR_6$, F, Cl, Br, $CH_3$, $OCH_3$, $NO_2$, $CF_3$, $SO_2NR_7R_8$, $S(O)_nR'_8$, $R_9\overset{O}{\overset{\|}{C}}$, $OSO_2R_{10}$ or $SO_2\underset{OCH_3}{\underset{|}{N}}(CH_3)$

in which Q is O, S or $-\underset{R_{13}}{\underset{|}{N}}-$ in which $R_{13}$ is H, $C_1$—$C_2$ alkyl or $OCH_3$,

$R_6$ is $C_1$—$C_4$ alkyl, $C_3$—$C_4$ alkenyl or $CH_3OCH_2CH_2$, or, when Q is O, then $R_6$ may also be $ClCH_2CH_2$, or $R_6$ and $R_{13}$ taken together form $-CH_2CH_2CH_2CH_2-$ or $-CH_2CH_2OCH_2CH_2-$,
$R_7$ and $R_8$, which may be the same or different, are $C_1$—$C_4$ alkyl provided that the total number of carbon atoms in $R_7$ and $R_8$ is less than or equal to five,
n is 0, 1 or 2,
$R'_8$ is $C_1$—$C_3$ alkyl,
$R_9$ is H or $C_1$—$C_4$ alkyl, and
$R_{10}$ is $C_1$—$C_4$ alkyl or $CF_3$,
$R_2$ is H, Cl, $CH_3$, $CF_3$, $NO_2$ or $OCH_3$, and
$R_3$ is H, F, Cl, Br, $CH_3$, $OCH_3$, $NO_2$, $CO_2R_{11}$ or $S(O)_nR_{12}$
in which $R_{11}$ is $C_1$—$C_4$ alkyl,
$R_{12}$ is $C_1$—$C_3$ alkyl, and
n is as defined above;
W is O or S;
$R_4$ is H or $CH_3$; and
$R_5$ is

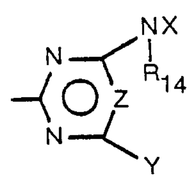

or

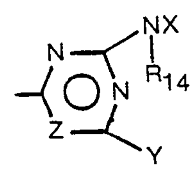

in which $R_{14}$ is H or $CH_3$,
Z is CH or N,
Y is F, Cl, Br, $C_1$—$C_2$ alkyl, $OCH_3$, $OCH_2CH_3$ or $CH_3OCH_2$, and
X is H,

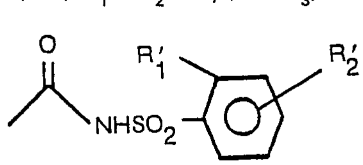

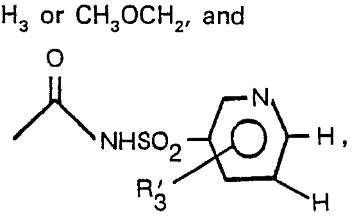

,

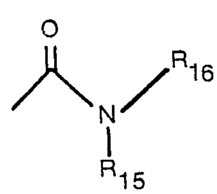

,

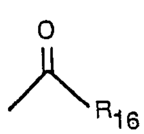

,

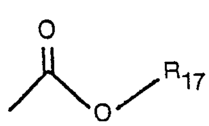

, $CH_3SO_2$ or $CF_3SO_2$,

in which $R_1'$ is 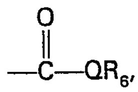, F, Cl, Br, $CH_3$, $OCH_3$, $NO_2$, $CF_3$, $SO_2NR_7R_8$, $S(O)_nR_8'$, 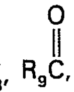, $OSO_2R_{10}$ or 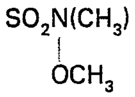

in which Q, $R_6$, $R_7$, $R_8$, $R_8'$, $R_9$, $R_{10}$ and n are as defined above,
$R_2'$ is H, Cl, $CH_3$, $CF_3$, $NO_2$ or $OCH_3$,
$R_3'$ is H, F, Cl, Br, $CH_3$, $OCH_3$, $NO_2$, $CO_2R_{11}$ or $S(O)_nR_{12}$
in which $R_{11}$, $R_{12}$ and n are defined above,
$R_{15}$ is H, $C_1$—$C_4$ alkyl, $C_3$—$C_4$ alkenyl or $OCH_3$,
$R_{16}$ is H, $C_1$—$C_6$ alkyl or cycloalkyl, phenyl, or phenyl optionally substituted by $CF_3$, F, $NO_2$, CN, 1—2 Cl, 1—2 Br, 1—2 $CH_3$ or 1—2 $OCH_3$, and
$R_{17}$ is $C_1$—$C_6$ alkyl or cycloalkyl, phenyl, or phenyl optionally substituted by $CF_3$, F, $NO_2$, CN, 1—2 Cl, 1—2 Br, 1—2 $CH_3$ or 1—2 $OCH_3$;
with the provisos that:

1) when X is H, then Y is other than $CH_3$, $OCH_3$ or $OCH_2CH_3$;
2) when Y is F, Cl or Br, then $R_5$ is

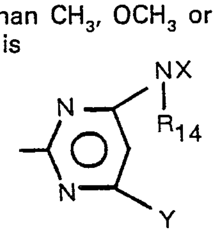

3) when $R_{15}$ is $OCH_3$, then $R_{16}$ is $CH_3$;
4) when $R_{13}$ is $OCH_3$, then $R_6$ is $CH_3$; and
5) when A is

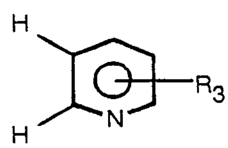

and $R_{14}$ is $CH_3$, then X is other than H;
and the agriculturally suitable salts thereof.
which comprises reacting a compound of formula

$$ASO_2NCW$$

(wherein A and W are as defined above) with a compound of formula

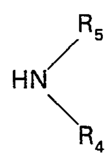

(wherein $R_4$ and $R_5$ are as defined above).

2. A process according to claim 1 wherein the reaction is effected in the presence of an inert aprotic organic solvent selected from acetonitrile, tetrahydrofuran and methylene chloride.

3. A process according to either of claims 1 or 2 wherein the reaction is effected at a temperature of from 25 to 81°C.

4. A process for the preparation of compounds of general formula I as defined in claim 1 in which W is O, $R_4$ is H, and $R_5$ is

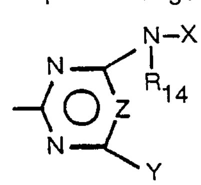

[in which $R_{14}$ is H; Z is CH; Y is Cl; and X is

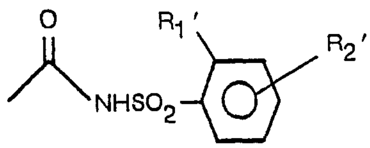

,

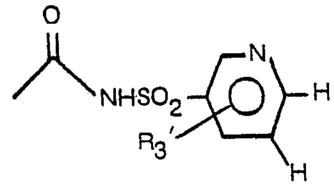

,

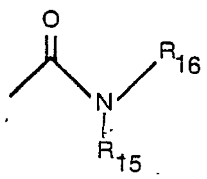

or

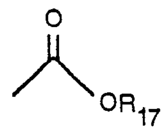

(in which $R_1'$, $R_2'$, $R_{15}$, $R_{16}$ and $R_{17}$ are as defined in claim 1)], which comprises reacting, in a first stage, a compound of formula

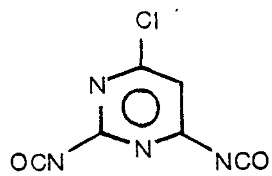

with a compound of formula

PH

[in which P is

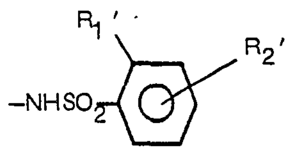

,

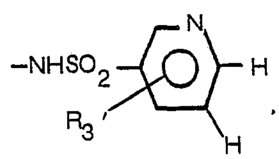

,

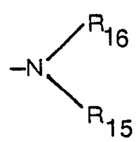

or

$-OR_{17}$

(in which $R_1'$, $R_2'$, $R_3'$, $R_{15}$, $R_{16}$ and $R_{17}$ are as defined in claim 1)], and, in a second stage, reacting the product of said first stage with a compound of formula

$$ASO_2NH_2$$

(in which A is as defined in claim 1).

5. A process according to claim 4 in which the product of the first stage is reacted with the compound of formula

$$ASO_2NH_2$$

without being isolated from the reaction medium in which it is formed.

6. A process according to either of claims 4 and 5 wherein the reactions of the first and second stages are effected in the presence of an inert aprotic solvent selected from acetonitrile, tetrahydrofuran and methylene chloride.

7. A process for the preparation of compounds of general formula I as defined in claim 1 in which W is O, $R_4$ is H and $R_5$ is

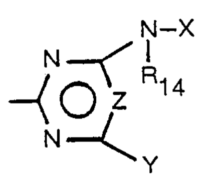

(in which $R_{14}$ is H, Z and Y are as defined in claim 1, and X is

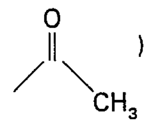

which comprises reacting a compound of formula

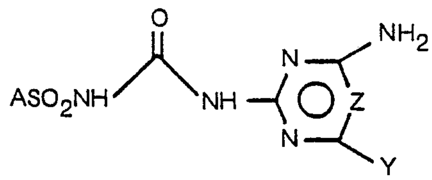 XIV

(in which A, Z and Y are as defined in claim 1) with acetic anhydride.

8. A process for the preparation of compounds of general formula I as defined in claim 1 in which W is O, $R_4$ is H and $R_5$ is

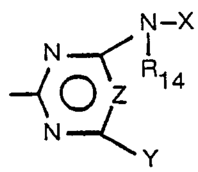

[in which $R_{14}$ is H, Z and Y are as defined in claim 1, and X is

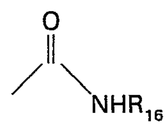

(in which $R_{16}$ is as defined in claim 1)], which comprises reacting a compound of formula XIV as defined in claim 7 with a compound of formula

$$R_{16}—NCO$$

(in which $R_{16}$ is as defined in claim 1).

9. A process for the preparation of compounds of general formula I as defined in claim 1 in which W is O, $R_4$ is H and $R_5$ is

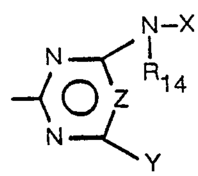

[in which $R_{14}$ is H, Z and Y are as defined in claim 1, and X is

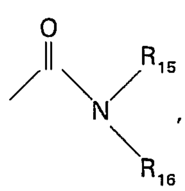 , 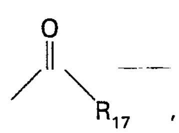 ,

$CH_3SO_2$ or $CF_3SO_2$ (in which $R_{15}$, $R_{16}$ and $R_{17}$ are as defined in claim 1)], which comprises reacting a compound of formula XIV as defined in claim 7 with a compound of formula

$$X—Cl$$

(in which X is as defined above).

10. A composition suitable for controlling the growth of undesired vegetation in a locus which comprises a compound of formula I as defined in claim 1 together with at least one surfactant, and/or solid or liquid diluent.

11. A method for controlling the growth of undesired vegetation in a locus which comprises applying to said locus an effective amount of a compound of formula I as defined in claim 1.

**Patentansprüche für die Vertragsstaaten: BE CH DE FR GB IT LI LU NL SE**

1. Verbindungen mit der allgemeinen Formel

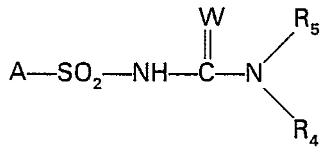

I

worin A die Bedeutung hat von

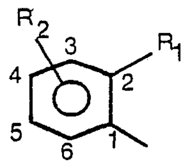

or

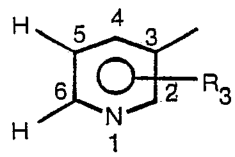

worin $R_1$ die Bedeutung hat von $-\overset{O}{\overset{\|}{C}}-QR_6$, F, Cl, Br, $CH_3$, $OCH_3$, $NO_2$, $CF_3$, $SO_2NR_7R_8$, $S(O)_nR_8'$, $R_9\overset{O}{\overset{\|}{C}}$, $OSO_2R_{10}$ oder $\underset{OCH_3}{SO_2N(CH_3)}$, worin Q die Bedeutung hat von O, S oder $\underset{R_{13}}{-N-}$

worin $R_{13}$ H, $C_1$—$C_2$-Alkyl oder $OCH_3$ ist,
$R_6$ $C_1$—$C_4$-Alkyl, $C_3$—$C_4$-Alkenyl oder $CH_3OCH_2CH_2$ ist, oder wenn Q O ist, dann kann $R_6$ auch $ClCH_2CH_2$ sein, oder können $R_6$ und $R_{13}$ zusammen $-CH_2CH_2CH_2CH_2-$ oder $-CH_2CH_2OCH_2CH_2-$ bilden,
$R_7$ und $R_8$, die gleich oder verschieden sein können, bedeuten $C_1$—$C_4$-Alkyl, vorausgesetzt daß die Gesamtanzahl der Kohlenstoffatome in $R_7$ und $R_8$ weniger als oder gleich fünf ist,
n 0, 1 oder 2 ist,
$R_8'$ $C_1$—$C_3$-Alkyl ist,
$R_9$ H oder $C_1$—$C_4$-Alkyl ist, und
$R_{10}$ $C_1$—$C_4$-Alkyl oder $CF_3$ ist,
$R_2$ H, Cl, $CH_3$, $CF_3$, $NO_2$ oder $OCH_3$ ist, und
$R_3$ H, F, Cl, Br, $CH_3$, $OCH_3$, $NO_2$, $CO_2R_{11}$ oder $S(O)_nR_{12}$ ist
worin $R_{11}$ $C_1$—$C_4$-Alkyl ist,
$R_{12}$ $C_1$—$C_3$-Alkyl ist, und
n wie vorstehend definiert ist;
W O oder S ist;
$R_4$ H oder $CH_3$ ist; und
$R_5$ die Bedeutung hat von

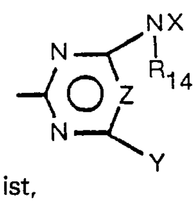

oder

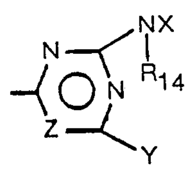

worin $R_{14}$ H oder $CH_3$ ist,
Z CH oder N ist,
Y F, Cl, Br, $C_1$—$C_2$-Alkyl, $OCH_3$, $OCH_2CH_3$ oder $CH_3OCH_2$ ist, und
X die Bedeutung hat von H,

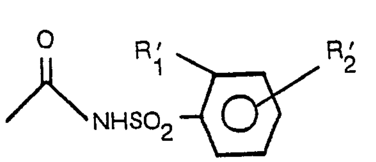

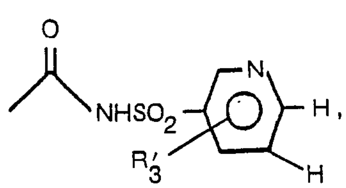

,

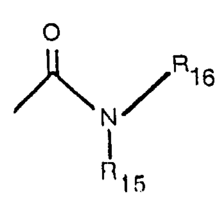

,

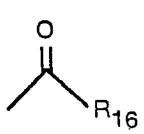

,

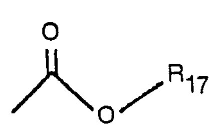

$CH_3SO_2$ oder $CF_3SO_2$,

worin $R_1'$ die Bedeutung hat von $-\overset{\overset{O}{\|}}{C}-QR_6$, F, Cl, Br, $CH_3$, $OCH_3$, $NO_2$, $CF_3$, $SO_2NR_7R_8$, $S(O)_nR_8'$, $R_9\overset{\overset{O}{\|}}{C}$, $OSO_2R_{10}$ oder $\underset{\underset{OCH_3}{|}}{SO_2N(CH_3)}$

worin Q, $R_6$, $R_7$, $R_8$, $R_8'$, $R_9$, $R_{10}$ und n wie vorstehend definiert sind,
$R_2'$ H, Cl, $CH_3$, $CF_3$, $NO_2$ oder $OCH_3$ ist,
$R_3'$ H, F, Cl, Br, $CH_3$, $OCH_3$, $NO_2$, $CO_2R_{11}$ oder $S(O)_nR_{12}$ ist,
worin $R_{11}$, $R_{12}$ und n wie vorstehend definiert sind,
$R_{15}$ H, $C_1$—$C_4$-Alkyl, $C_3$—$C_4$-Alkenyl oder $OCH_3$ ist,
$R_{16}$ H, $C_1$—$C_6$-Alkyl oder Cycloalkyl, Phenyl, oder Phenyl gegebenenfalls substituiert durch $CF_3$, F, $NO_2$, CN, 1—2 Cl, 1—2 Br, 1—2 $CH_3$ oder 1—2 $OCH_3$ ist, und
$R_{17}$ $C_1$—$C_6$-Alkyl oder Cycloalkyl, Phenyl, oder Phenyl gegebenenfalls substituiert durch $CF_3$, F, $NO_2$, CN, 1—2 Cl, 1—2 Br, 1—2 $CH_3$ oder 1—2 $OCH_3$ ist;

mit den Maßgaben daß:
1) wenn X H ist, dann ist Y unterschiedlich von $CH_3$, $OCH_3$ oder $OCH_2CH_3$;
2) wenn Y, F, Cl oder Br ist, dann ist $R_5$

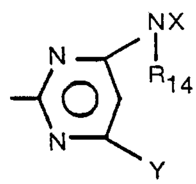

3) wenn $R_{15}$ $OCH_3$ ist, dann ist $R_{16}$ $CH_3$;
4) wenn $R_{13}$ $OCH_3$ ist, dann ist $R_6$ $CH_3$; und
5) wenn A die Bedeutung hat von

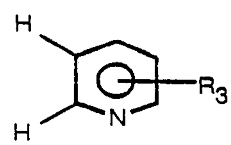

und $R_{14}$ $CH_3$ ist, dann ist X von H unterschiedlich;
und die landwirtschaftlich brauchbaren Salze davon.

2. Verbindungen nach Anspruch 1, worin W O ist und $R_4$ H ist.

3. Verbindungen nach Anspruch 2, worin $R_1$ und/oder $R_1'$ die Bedeutung haben von

$-\overset{\overset{O}{\|}}{C}-QR_6$, $NO_2$, Cl, $SO_2NR_7R_8$, $\underset{\underset{CH_3}{|}}{SO_2N(OCH_3)}$, $S(O)_nR_8'$ oder $OSO_2R_{10}$.

4. Verbindungen nach Anspruch 3, worin $R_5$ die Bedeutung hat von

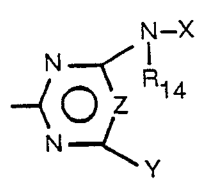

5. Verbindungen nach Anspruch 4, worin $R_1'$ gleich ist wie $R_1$, $R_2'$ gleich ist wie $R_2$, $R_3'$ gleich ist wie $R_3$ und $R_3$ sich in der 2-Stellung des Pyridinrings befindet.

6. Verbindungen nach Anspruch 5, worin $R_{15}$ $C_1$—$C_2$-Alkyl, $CH_2$—$CH{=}CH_2$, $OCH_3$ oder H ist; und $R_{16}$ H, $C_1$—$C_3$-Alkyl, Phenyl, oder Phenyl gegebenenfalls substituiert durch $CF_3$, $NO_2$, 1—2 Cl, $CH_3$ oder $OCH_3$ ist.

7. Verbindungen nach Anspruch 6, worin $R_{17}$ $C_1$—$C_3$-Alkyl, Phenyl, oder Phenyl gegebenenfalls substituiert durch Cl, Br, $CH_3$ oder $OCH_3$ ist.

8. Verbindungen nach Anspruch 7, worin $R_2$ H ist.

9. Verbindungen nach Anspruch 8, worin $R_6$ $C_1$—$C_3$-Alkyl oder $CH_2CH{=}CH_2$ ist; $R_{13}$ $CH_3$ ist; $R_1$ $NO_2$, $SO_2N(R_7,R_8)_2$, $SO_2N(CH_3)$,

$OCH_3$

$SO_2R_8'$ oder $OSO_2CH_3$ ist; und $R_3$ Cl oder $SO_2CH_3$ ist; vorausgesetzt daß die Gesamtanzahl der Kohlenstoffatome in ($R_7$, $R_8$) nicht größer als vier ist.

10. Verbindungen nach Anspruch 9, worin Z CH ist.

11. Verbindungen nach Anspruch 10, worin X H,

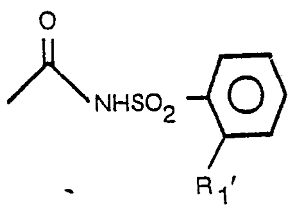
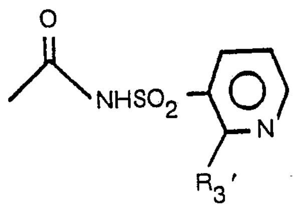
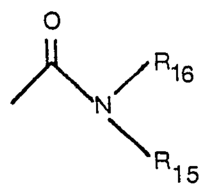

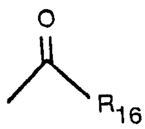
,
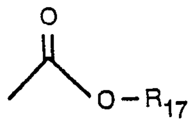
, $CH_3SO_2^-$ oder $CF_3SO_2$ ist.

12. Verbindungen nach Anspruch 11, worin X

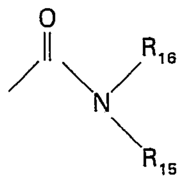
ist,

worin $R_{15}$ H, $C_1$—$C_3$-Alkyl oder $OCH_3$ ist, und $R_{16}$ H oder $C_1$—$C_3$-Alkyl ist.

13. Verbindungen nach Anspruch 11, worin X

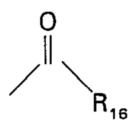

ist, worin $R_{16}$ $C_1$—$C_3$-Alkyl ist.

14. Verbindungen nach Anspruch 11, worin X

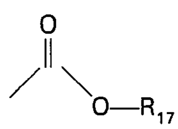

ist, worin $R_{17}$ $C_1$—$C_3$-Alkyl ist.

15. Verbindung nach Anspruch 1, nämlich Methyl-2-{[(4-amino-6-chlorpyrimidin-2-yl)-aminocarbonyl]aminosulfonyl}-benzoat.

16. Verbindung nach Anspruch 1, nämlich Dimethyl-2,2′-{6-chlorpyrimidin-4,2-diyl-bis-[(aminocarbonyl)-aminosulfonyl]}-bis-[benzoat].

17. Verbindung nach Anspruch 1, nämlich N-{4-[((2-CHlorphenyl)-sulfonylamino)-carbonylamino]-6-methyl-1,3,5-triazin-2-yl}-acetamid.

18. Verbindung nach Anspruch 1, nämlich Methyl-2-{[(4-acetylamino)-6-methyl-1,3,5-triazin-2-yl)-aminocarbonyl]-aminosulfonyl}-benzoat.

19. Zusammensetzung geeignet zur Bekämpfung des Wachstums underwünschter Vegetation, enthaltend eine wirksame Menge einer Verbindung nach einem der Ansprüche 1 bis 18, zusammen mit mindestens einem oberflächenaktiven Mittel und/oder festen oder flüssigen Verdünnungsmittel.

20. Verfahren zur Bekämpfung des Wachstums unerwünschter Vegetation an einem Ort, durch Auftragen auf den Ort, von einer wirksamen Menge einer Verbindung gemäß einem der Ansprüche 1 bis 19.

**Patentansprüche für den Vertragsstaat: AT**

1. Verfahren zur Herstellung von Verbindungen der allgemeinen Formel

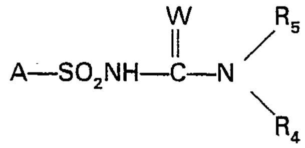

I

worin A die Bedeutung hat von

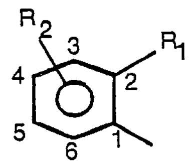

oder

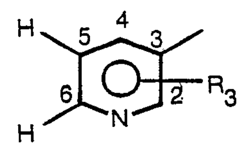

worin $R_1$ die Bedeutung hat von $\overset{\overset{O}{\|}}{C}—QR_6$, F, Cl, Br, $CH_3$, $OCH_3$, $NO_2$, $CF_3$, $SO_2NR_7R_8$, $S(O)_nR'_8$, $R_9\overset{\overset{O}{\|}}{C}$, $OSO_2R_{10}$ oder $\underset{OCH_3}{SO_2N(CH_3)}$, worin Q die Bedeutung hat von O, S oder $\underset{R_{13}}{—N—}$

worin $R_{13}$ H, $C_1$—$C_2$-Alkyl oder $OCH_3$ ist,
$R_6$ $C_1$—$C_4$-Alkyl, $C_3$—$C_4$-Alkenyl oder $CH_3OCH_2CH_2$ ist, oder wenn Q O ist, dann kann $R_6$ auch $ClCH_2CH_2$ sein, oder können $R_6$ und $R_{13}$ zusammen —$CH_2CH_2CH_2CH_2$— oder —$CH_2CH_2OCH_2CH_2$— bilden,
$R_7$ und $R_8$, die gleich oder verschieden sein können, bedeuten $C_1$—$C_4$-Alkyl, vorausgesetzt daß die Gesamtanzahl der Kohlenstoffatome in $R_7$ und $R_8$ weniger als oder gleich fünf ist,
n 0, 1 oder 2 ist,
$R'_8$ $C_1$—$C_3$-Alkyl ist,
$R_9$ H oder $C_1$—$C_4$-Alkyl ist, und
$R_{10}$ $C_1$—$C_4$-Alkyl oder $CF_3$ ist,
$R_2$ H, Cl, $CH_3$, $CF_3$, $NO_2$ oder $OCH_3$ ist, und
$R_3$ H, F, Cl, Br, $CH_3$, $OCH_3$, $NO_2$, $CO_2R_{11}$ oder $S(O)_nR_{12}$ ist
worin $R_{11}$ $C_1$—$C_4$-Alkyl ist,
$R_{12}$ $C_1$—$C_3$-Alkyl ist, und
n wie vorstehend definiert ist;
W O oder S ist;
$R_4$ H oder $CH_3$ ist; und
$R_5$ die Bedeutung hat von

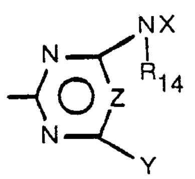

oder

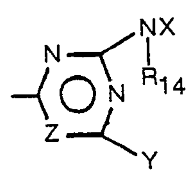

worin $R_{14}$ H oder $CH_3$ ist,
Z CH oder N ist,
Y F, Cl, Br, $C_1$—$C_2$-Alkyl, $OCH_3$, $OCH_2CH_3$ oder $CH_3OCH_2$ ist, und
X die Bedeutung hat von H,

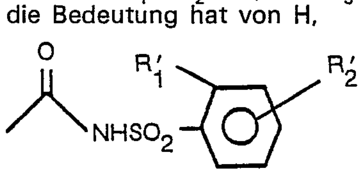

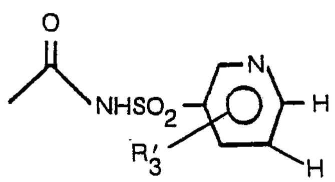

,

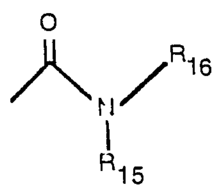

,

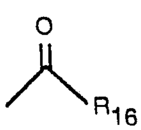

,

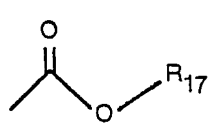

, $CH_3SO_2$ oder $CF_3SO_2$,

worin $R_1'$ die Bedeutung hat von 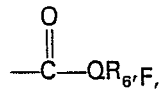, F, Cl, Br, $CH_3$, $OCH_3$, $NO_2$, $CF_3$, $SO_2NR_7R_8$, $S(O)_nR_8'$,

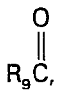, $OSO_2R_{10}$ oder 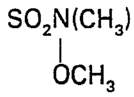

worin Q, $R_6$, $R_7$, $R_8$, R, $R_9$, $R_{10}$ und n wie vorstehend definiert sind,
$R_2'$ H, Cl, $CH_3$, $CF_3$, $NO_2$ oder $OCH_3$ ist,
$R_3'$ H, F, Cl, Br, $CH_3$, $OCH_3$, $NO_2$, $CO_2R_{11}$ oder $S(O)_nR_{12}$ ist,
worin $R_{11}$, $R_{12}$ und n wie vorstehend definiert sind,
$R_{15}$ H, $C_1$—$C_4$-Alkyl, $C_3$—$C_4$-Alkenyl oder $OCH_3$ ist,
$R_{16}$ H, $C_1$—$C_6$-Alkyl oder Cycloalkyl, Phenyl, oder Phenyl gegebenenfalls substituiert durch $CF_3$, F, $NO_2$, CN, 1—2 Cl, 1—2 Br, 1—2 $CH_3$ oder 1—2 $OCH_3$ ist, und
$R_{17}$ $C_1$—$C_6$-Alkyl oder Cycloalkyl, Phenyl, oder Phenyl gegebenenfalls substituiert durch $CF_3$, F, $NO_2$, CN, 1—2 Cl, 1—2 Br, 1—2 $CH_3$ oder 1—2 $OCH_3$ ist;

mit den Maßgaben daß:
1) wenn X H ist, dann ist Y unterschiedlich von $CH_3$, $OCH_3$ oder $OCH_2CH_3$;
2) wenn Y, F, Cl oder Br ist, dann ist $R_5$

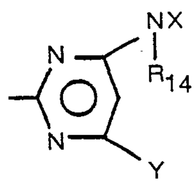 ;

3) wenn $R_{15}$ $OCH_3$ ist, dann ist $R_{16}$ $CH_3$;
4) wenn $R_{13}$ $OCH_3$ ist, dann ist $R_6$ $CH_3$; und
5) wenn A die Bedeutung hat von

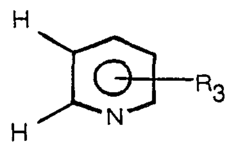

und $R_{14}$ $CH_3$ ist, dann ist X von H unterschiedlich;

und der landwirtschaftlich brauchbaren Salze davon, bei dem eine Verbindung der Formel

$$ASO_2NCW$$

(worin A und W wie vorstehend definiert sind) mit einer Verbindung der Formel

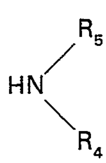

(worin $R_4$ und $R_5$ wie vorstehend definiert sind) umgesetzt wird.

2. Verfahren nach Anspruch 1, bei dem die Reaktion in Anwesenheit eines inerten aprotischen organischen Lösungsmittels durchgeführt wird, das ausgewählt ist aus Acetonitril, Tetrahydrofuran und Methylenchlorid.

3. Verfahren nach einem der Ansprüche 1 und 2, bei dem die Reaktion bei einer Temperatur von 25 bis 81°C durchgeführt wird.

4. Verfahren zur Herstellung von Verbindungen der allgemeinen Formel I, wie in Anspruch 1 definiert, worin W O ist, $R_4$ H ist und $R_5$ die Bedeutung hat von

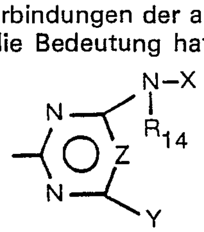 .

[worin $R_{14}$ H ist, Z CH ist; Y Cl ist; und X die Bedeutung hat von

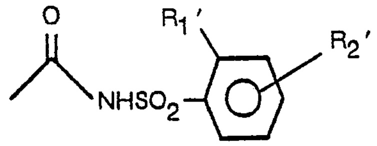 , 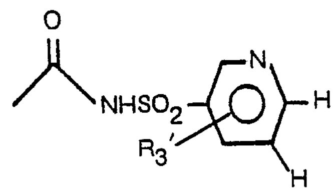 ,

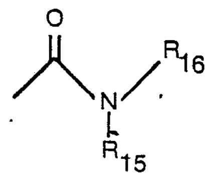 oder 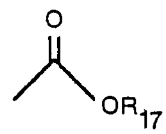

(worin $R_1'$, $R_2'$, $R_3'$, $R_{15}$, $R_{16}$ und $R_{17}$ wie in Anspruch 1 definiert sind)], durch Reaktion in einer ersten Stufe von einer Verbindung der Formel

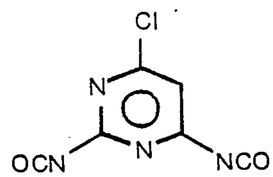

mit einer Verbindung der Formel

PH

[worin P die Bedeutung hat von

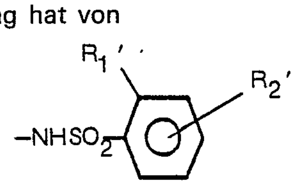 , 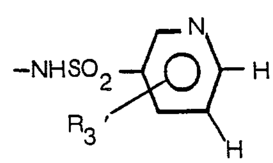 ,

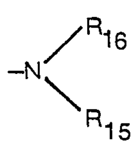 oder $-OR_{17}$

(worin $R_1'$, $R_2'$, $R_3'$, $R_{15}$, $R_{16}$ und $R_{17}$ wie in Anspruch 1 definiert sind)] und in einer zweiten Stufe, Reaktion des Produkts der ersten Stufe mit einer Verbindung der Formel

$$ASO_2NH_2$$

(worin A wie in Anspruch definiert ist).

5. Verfahren nach Anspruch 4, bei dem das Produkt der ersten Stufe umgesetzt wird mit der Verbindung der Formel

$$ASO_2NH_2$$

ohne Isolieren aus dem Reaktionsmedium in dem es gebildet wird.

6. Verfahren nach einem der Ansprüche 4 und 5, bei dem die Reaktionen der ersten und zweiten Stufen in Anwesenheit eines inerten aprotischen Lösungsmittels durchgeführt wird, das ausgewählt wird aus Acetonitril, Tetrahydrofuran und Methylenchlorid.

7. Verfahren zur Herstellung von Verbindungen der allgemeinen Formel I, wie in Anspruch 1 definiert, worin W O ist, $R_4$ H ist und $R_5$

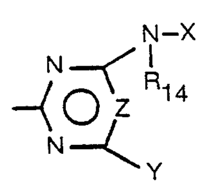 ist,

(worin $R_{14}$ H ist, Z und Y wie in Anspruch 1 definiert sind und X

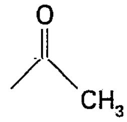

ist), durch Reaktion einer Verbindung der Formel

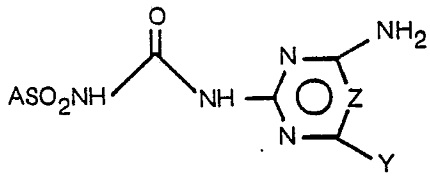

XIV

(worin A, Z und Y wie in Anspruch 1 definiert sind) mit Essigsäureanhydrid.

8. Verfahren zur Herstellung von Verbindung der allgemeinen Formel I, wie in Anspruch 1 definiert, worin W O ist, $R_4$ H ist und $R_5$

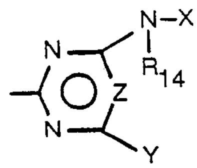

ist,

[worin $R_{14}$ H ist, Z und Y wie in Anspruch 1 definiert sind un X

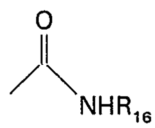

ist (worin $R_{16}$ wie in Anspruch 1 definiert ist)], durch Reaktion einer Verbindung der Formel XIV, wie in Anspruch 7 definiert, mit einer Verbindung der Formel

$$R_{16}—NCO$$

(worin $R_{16}$ wie in Anspruch 1 definiert ist).

9. Verfahren zur Herstellung von Verbindung der allgemeinen Formel I, wie in Anspruch 1 definiert, worin W O ist, $R_4$ H ist und $R_5$

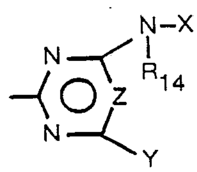

ist,

[worin $R_{14}$ H ist, Z und Y wie in Anspruch 1 definiert sind und X

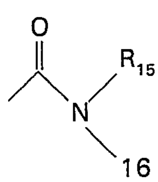
,
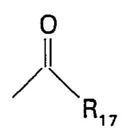
,

$CH_3SO_2$ oder $CF_3SO_2$ ist (worin $R_{15}$, $R_{16}$ und $R_{17}$ wie in Anspruch 1 definiert sind)], durch Reaktion einer Verbindung der Formel XIV, wie in Anspruch 7 definiert, mit einer Verbindung der Formel

$$X—Cl$$

(worin X wie vorstehend definiert ist).

10. Zusammensetzung geeignet zur Bekämpfung des Wachstums unerwünschter Vegetation an einem Ort, die eine Verbindung der Formel I, wie in Anspruch 1 definiert, zusammen mit mindestens einem oberflächenaktiven Mittel, und/oder festen oder flüssigen Verdünnungsmittel enthält.

11. Verfahren zur Bekämpfung des Wachstums unerwünschter Vegetation an einem Ort, durch Auftrag auf den Ort von einer wirksamen Menge einer Verbindung der Formel I, wie in Anspruch 1 definiert.

**Revendications pour les Etats contractants: BE CH DE FR GB IT LI LU NL SE**

1. Composés de formule générale:

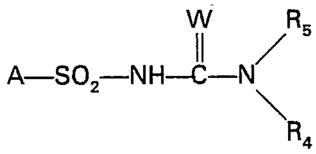

I

dans laquelle
A est:

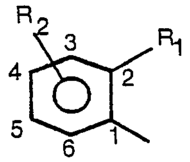

ou

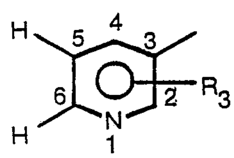

où $R_1$ est $-\overset{O}{\overset{\|}{C}}-QR_6$, F, Cl, Br, $CH_3$, $OCH_3$, $NO_2$, $CF_3$, $SO_2NR_7R_8$, $S(O)_nR'_8$, $R_9\overset{O}{\overset{\|}{C}}$,

$OSO_2R_{10}$ ou $\underset{OCH_3}{SO_2N(CH_3)}$, où Q est O, S ou $\underset{R_{13}}{-N-}$

où $R_{13}$ est H, un groupe alkyle en $C_1$ à $C_2$ ou $OCH_3$,
$R_6$ est un groupe alkyle en $C_1$ à $C_4$, un groupe alcényle en $C_1$ à $C_4$ ou $CH_3OCH_2CH_2$ ou, lorsque Q est O, $R_6$ peut aussi être $ClCH_2CH_2$, ou bien $R_6$ et $R_{13}$ pris ensemble forment $-CH_2CH_2CH_2CH_2-$ ou $-CH_2CH_2OCH_2CH_2-$,
$R_7$ et $R_8$, qui peuvent être semblables ou différents, sont des groupes alkyle en $C_1$ à $C_4$, étant entendu que le nombre total d'atomes de carbone dans $R_7$ et $R_8$ est inférieur ou égal à 5,
*n* vaut 0, 1 ou 2,
$R'_8$ est un groupe alkyle en $C_1$ à $C_3$,
$R_9$ est H ou un groupe alkyle en $C_1$ à $C_4$, et
$R_{10}$ est un groupe alkyle en $C_1$ à $C_4$ ou $CF_3$,
$R_2$ est H, Cl, $CH_3$, $CF_3$, $NO_2$ ou $OCH_3$, et
$R_3$ est H, F, Cl, Br, $CH_3$, $OCH_3$, $NO_2$, $CO_2R_{11}$ ou $S(O)_nR_{12}$,
où $R_{11}$ est un groupe alkyle en $C_1$ à $C_4$,
$R_{12}$ est un groupe alkyle en $C_1$ à $C_3$, et
*n* est tel que défini plus haut;
W est O ou S;
$R_4$ est H ou $CH_3$ et
$R_5$ est

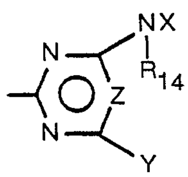

ou

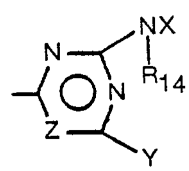

où $R_{14}$ est H ou $CH_3$,
Z est CH ou N,
Y est F, Cl, Br, un groupe alkyle en $C_1$ à $C_2$, $OCH_3$, $OCH_2CH_3$ ou $CH_3OCH_2$, et
X est

H, 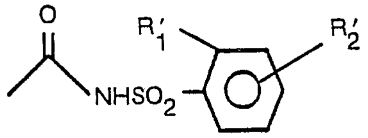 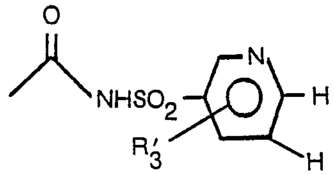 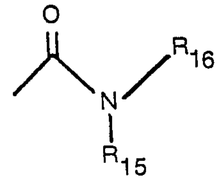 ,

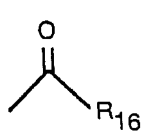 , 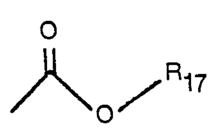 , $CH_3SO_2$ ou $CF_3SO_2$

où $R_1'$ est 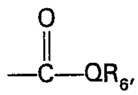, F, Cl, Br, $CH_3$, $OCH_3$, $NO_2$, $CF_3$, $SO_2NR_7R_8$, $S(O)_nR_8'$, 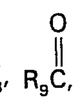, $OSO_2R_{10}$ ou 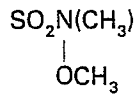

où Q, $R_6$, $R_7$, $R_8$, $R_8'$, $R_9$, $R_{10}$ et *n* sont tels que définis plus haut,
$R_2'$ est H, Cl, $CH_3$, $CF_3$, $NO_2$ ou $OCH_3$,
$R_3'$ est H, F, Cl, Br, $CH_3$, $OCH_3$, $NO_2$, $CO_2R_{11}$ ou $S(O)_nR_{12}$,
où $R_{11}$, $R_{12}$ et *n* sont tels que définis plus haut,
$R_{15}$ est H, un groupe alkyle en $C_1$ à $C_4$, un groupe alcényle en $C_3$ à $C_4$ ou $OCH_3$,
$R_{16}$ est H, un groupe alkyle en $C_1$ à $C_6$ ou cycloalkyle, phényle ou phényle facultativement substitué par $CF_3$, F, $NO_2$, CH, 1 à 2 Cl, 1 à 2 Br, 1 à 2 $CH_3$ ou 1 à 2 $OCH_3$, et
$R_{17}$ est un groupe alkyle en $C_1$ à $C_6$ ou cycloalkyle, phényle ou phényle facultativement substitué par $CF_3$, F, $NO_2$, CN, 1 à 2 Cl, 1 à 2 Br, 1 à 2 $CH_3$ ou 1 à 2 $OCH_3$, étant entendu que:

1) quand X est H, Y n'est pas $CH_3$ $OCH_3$ ou $OCH_2CH_3$;
2) quand Y est F, Cl ou Br, $R_5$ est

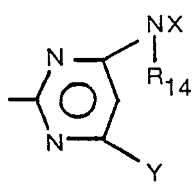 ;

3) quand $R_{15}$ est $OCH_3$, $R_{16}$ est $CH_3$;
4) quand $R_{13}$ est $OCH_3$, $R_6$ est $CH_3$; et
5) quand A est

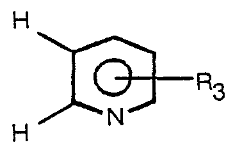

et $R_{14}$ est $CH_3$, X n'est pas H,
ainsi que leurs sels utilisables en agriculture.

2. Composés selon la revendication 1, dans lesquels W est O et $R_4$ est H.

3. Composés selon la revendication 2, dans lesquels $R_1$ et/ou $R_1'$ sont:

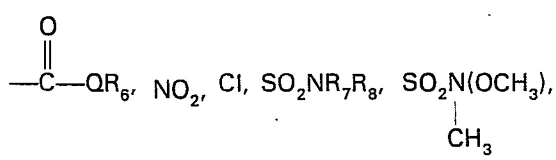

$S(O)_nR_8'$ ou $OSO_2R_{10}$.

4. Composés selon la revendication 3, dans lesquels $R_5$ est

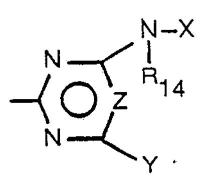

5. Composés selon la revendication 4, dans lesquels $R_1'$ est semblable à $R_1$, $R_2'$ est semblable à $R_2$, $R_3'$ est semblable à $R_3$, et $R_3$ est en position 2 du noyau pyridine.

6. Composés selon la revendication 5, dans lesquels $R_{15}$ est un groupe alkyle en $C_1$ à $C_2$, $CH_2$—$CH{=}CH_2$, $OCH_3$ ou H; et $R_{16}$ est H, un groupe alkyle en $C_1$ à $C_3$, phényle ou phényle facultativement substitué par $CF_3$, $NO_2$, 1 à 2 Cl, $CH_3$ ou $OCH_3$.

7. Composés selon la revendication 6, dans lesquels $R_{17}$ est un groupe alkyle en $C_1$ à $C_3$, phényle ou phényle facultativement substitué par Cl, Br, $CH_3$ ou $OCH_3$.

8. Composés selon la revendication 7, dans lesquels $R_2$ est H.

9. Composés selon la revendication 8, dans lesquels $R_6$ est un groupe alkyle en $C_1$ à $C_3$ ou $CH_2CH{=}CH_2$; $R_{13}$ est $CH_3$; $R_1$ est $NO_2$, $SO_2N(R_7, R_8)_2$, $SO_2N(CH_3)$,
$\quad | \quad OCH_3$
$SO_2R'_8$ ou $OSO_2CH_3$;
et $R_3$ est Cl ou $SO_2CH_3$, étant entendu que le nombre total d'atomes de carbone dans $(R_7, R_8)$ n'est pas supérieur à 4.

10. Composés selon la revendication 9, dans lesquels Z est CH.

11. Composés selon la revendication 10, dans lesquels X est H,

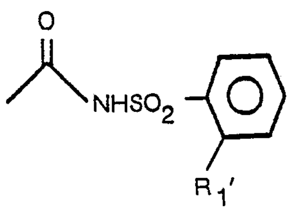
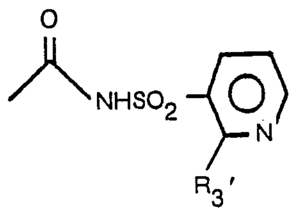
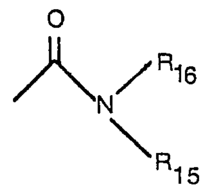

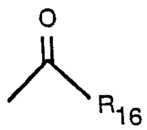 , 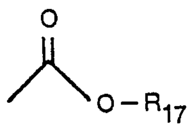 , $CH_3SO_2$ ou $CF_3SO_2$.

12. Composés selon la revendication 1, dans lesquels X est

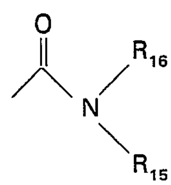 ,

où $R_{15}$ est H, un groupe alkyle en $C_1$ à $C_3$ ou $OCH_3$, et $R_{16}$ est H ou un groupe alkyle en $C_1$ à $C_3$.

13. Composés selon la revendication 11, dans lesquels X est

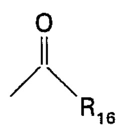

dans lequel $R_{16}$ est un groupe alkyle en $C_1$ à $C_3$.

14. Composés selon la revendication 11, dans lesquels X est

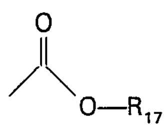

dans lequel $R_{17}$ est un groupe alkyle en $C_1$ à $C_3$.

15. Composé selon la revendication 1, qui est le 2-{[(4-amino-6-chloropyrimidin-2-yl)-aminocarbonyl]-aminosulfonyl}-benzoate de méthyle.

16. Composé selon la revendication 1, qui est le 2,2'-{6-chloropyrimidin-4,2-diyl-bis-[(aminocarbonyl)-aminosulfonyl]}-dibenzoate de diméthyle.

17. Composé selon la revendication 1, qui est la N-{4-[((2-chlorophényl)-sulfonylamino)-carbonylamino]-6-méthyl-1,3,5-triazin-2-yl}-acétamide.

18. Composé selon la revendication 1, qui est le 2-{[(4-(acétylamino)-6-méthyl-1,3,5-triazin-2-yl)-aminocarbonyl]-aminosulfonyl}-benzoate de méthyle.

19. Composition propre à maîtriser la croissance de végétation indésirable, qui comprend une quantité efficace d'un composé selon l'une quelconque des revendications 1 à 18, ainsi qu'au moins un agent tensio-actif et/ou un diluant solide ou liquide.

20. Procédé pour maîtriser la croissance de végétation indésirable en un lieu, qui consiste à appliquer à ce lieu une quantité efficace d'un composé selon l'une quelconque des revendications 1 à 19.

**Revendications pour l'Etat contractant: AT**

1. Procédé de préparation de composés de formule générale:

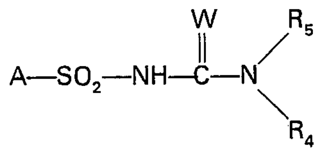

I

dans laquelle A est:

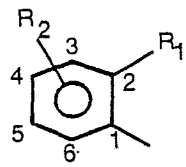

ou

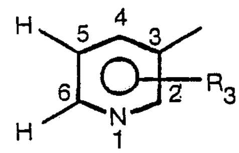

où $R_1$ est $-\overset{O}{\overset{\|}{C}}-QR_6$, F, Cl, Br, $CH_3$, $OCH_3$, $NO_2$, $CF_3$, $SO_2NR_7R_8$, $S(O)_nR_8'$, $R_9\overset{O}{\overset{\|}{C}}$, $OSO_2R_{10}$ ou $\underset{\underset{OCH_3}{|}}{SO_2N(CH_3)}$, où Q est O, S ou $-\underset{\underset{R_{13}}{|}}{N}-$

où $R_{13}$ est H, un groupe alkyle en $C_1$ à $C_2$ ou $OCH_3$,
$R_6$ est un groupe alkyle en $C_1$ à $C_4$, un groupe alcényle en $C_3$ à $C_4$ $CH_3OCH_2CH_2$, lorsque Q est O, $R_6$ peut aussi être $ClCH_2CH_2$, ou bien $R_6$ et $R_{13}$ pris ensemble forment $-CH_2CH_2CH_2CH_2-$ ou $-CH_2CH_2OCH_2CH_2-$,
$R_7$ et $R_8$, qui peuvent être semblables ou différents, sont des groupes alkyle en $C_1$ à $C_4$, étant entendu que le nombre total d'atomes de carbone dans $R_7$ et $R_8$ est inférieur ou égal à 5,
*n* vaut 0, 1 ou 2,
$R_8'$ est un groupe alkyle en $C_1$ à $C_3$,
$R_9$ est H ou un groupe alkyle en $C_1$ à $C_4$, et
$R_{10}$ est un groupe alkyle en $C_1$ à $C_4$ ou $CF_3$,
$R_2$ est H, Cl, $CH_3$, $CF_3$, $NO_2$ ou $OCH_3$, et
$R_3$ est H, F, Cl, Br, $CH_3$, $OCH_3$, $NO_2$, $CO_2R_{11}$ ou $S(O)_nR_{12}$,
où $R_{11}$ est un groupe alkyle en $C_1$ à $C_4$,
$R_{12}$ est un groupe alkyle en $C_1$ à $C_3$, et
*n* est tel que défini plus haut;
W est O ou S;
$R_4$ est H ou $CH_3$ et
$R_5$ est

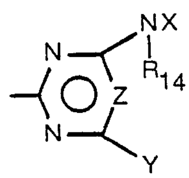

ou

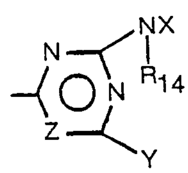

où $R_{14}$ est H ou $CH_3$,
Z est CH ou N,
Y est F, Cl, Br, un groupe alkyle en $C_1$ à $C_2$, $OCH_3$, $OCH_2\ CH_3$ ou $CH_3OCH_2$, et
X est

H, 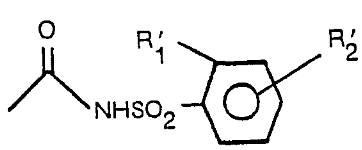 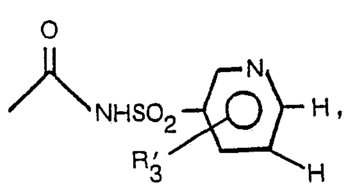 , 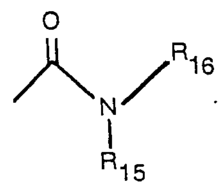 ,

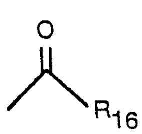 , 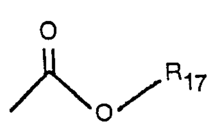 , $CH_3SO_2$ ou $CF_3SO_2$

où $R_1'$ est 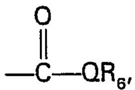, F, Cl, Br, $CH_3$, $OCH_3$, $NO_2$, $CF_3$, $SO_2NR_7R_8$, $S(O)_nR_8'$, 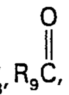,

$OSO_2R_{10}$ ou 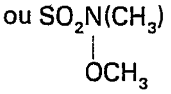

où Q, $R_6$, $R_7$, $R_8$, $R_8'$, $R_9$, $R_{10}$ et *n* sont tels que définis plus haut,
$R_2'$ est H, Cl, $CH_3$, $CF_3$, $NO_2$ ou $OCH_3$,
$R_3'$ est H, F, Cl, Br, $CH_3$, $OCH_3$, $NO_2$, $CO_2R_{11}$ ou $S(O)_nR_{12}$,
où $R_{11}$, $R_{12}$ et *n* sont tels que définis plus haut,
$R_{15}$ est H, un groupe alkyle en $C_1$ à $C_4$, un groupe alcényle en $C_3$ à $C_4$ ou $OCH_3$,
$R_{16}$ est H, un groupe alkyle en $C_1$ à $C_6$ ou cycloalkyle, phényle ou phényle facultativement substitué par $CF_3$, F, $NO_2$, CH, 1 à 2 Cl, 1 à 2 Br, 1 à 2 $CH_3$ ou 1 à 2 $OCH_3$, et
$R_{17}$ est un groupe alkyle en $C_1$ à $C_6$ ou cycloalkyle, phényle ou phényle facultativement substitué par $CF_3$, F, $NO_2$, CN, 1 à 2 Cl, 1 à 2 Br, 1 à 2 $CH_3$ ou 1 à 2 $OCH_3$, étant entendu que:
1) quand X est H, Y n'est pas $CH_3$ $OCH_3$ ou $OCH_2CH_3$;
2) quand Y est F, Cl ou Br, $R_5$ est

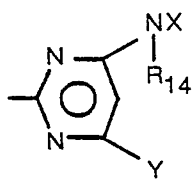 ;

3) quand $R_{15}$ est $OCH_3$, $R_{16}$ est $CH_3$;
4) quand $R_{13}$ est $OCH_3$, $R_6$ est $CH_3$; et
5) quand A est

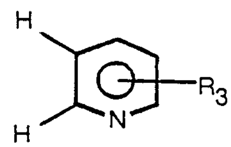

et $R_{14}$ est $CH_3$, X n'est pas H,
ainsi que leurs sels utilisables en agriculture qui consiste à faire réagir un composé de formule

$$ASO_2NCW$$

(dans laquelle A et W sont tels que définis plus haut) sur un composé de formule:

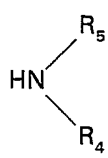

(dans laquelle $R_4$ et $R_5$ sont tels que définis plus haut).

2. Procédé selon la revendication 1, dans lequel on conduit la réaction en présence d'un solvant organique aprotique inerte choisi parmi l'acétonitrile, le tétrahydrofuranne et le chlorure de méthylène.

3. Procédé selon l'une des revendications 1 et 2, dans laquel on conduit la réaction à une température de 25 à 81°C.

4. Procédé de préparation de composés de formule générale I telle que définie à la revendication 1, dans lesquels W est O, $R_4$ est H, et $R_5$ est

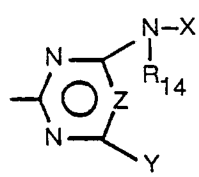

[dans lequel $R_{14}$ est H, Z est CH, Y est Cl et X est

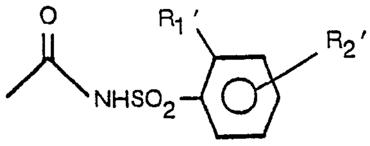

,

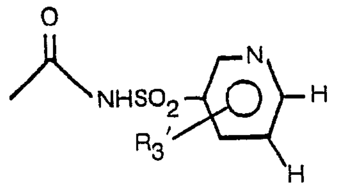

,

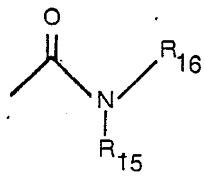

ou

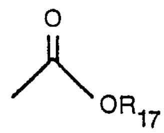

(où $R_1'$, $R_2'$, $R_3'$, $R_{15}$, $R_{16}$ et $R_{17}$ sont tels que définis à la revendication 1)], qui consiste à faire réagir, dans un premier stade, un composé de formule:

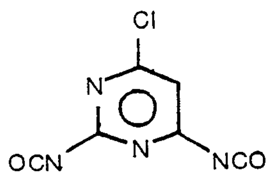

avec un composé de formule

PH

[dans lequel P est

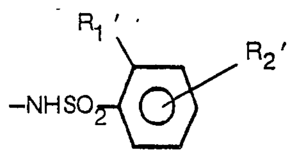

,

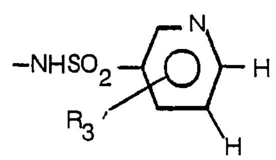

,

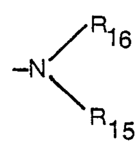

ou

$-OR_{17}$

(où $R_1'$, $R_2'$, $R_3'$, $R_{15}$, $R_{16}$ et $R_{17}$ sont tels que définis à la revendication 1)] et, dans un deuxième stade, à faire réagir le produit de ce premier stade avec un composé de formule:

$$ASO_2NH_2$$

(où A est tel que défini à la revendication 1).

5. Procédé selon la revendication 4, dans lequel on fait réagir le produit du premier stade avec le composé de formule

$$ASO_2NH_2$$

sans l'isoler du milieu réactionnel dans lequel il s'est formé.

6. Procédé selon l'une des revendications 4 et 5, dans lequel on conduit les réactions des premier et deuxième stades en présence d'un solvant aprotique inerte choisi parmi l'acétronitrile, le tétrahydrofuranne et le chlorure de méthylène.

7. Procédé de préparation de composés de formule générale I telle que définie à la revendication 1, dans laquelle W est O, $R_4$ est H et $R_5$ est

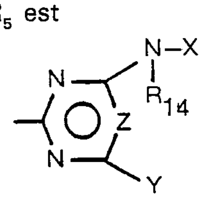

.

(où $R_{14}$ est H, Z et Y sont tels que définis à la revendication 1, et X est

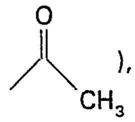

),

qui consiste à faire réagir un composé de formule

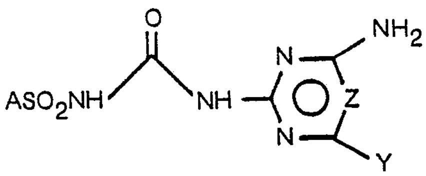

XIV

(où A, Z et Y sont tels que définis à la revendication 1) avec de l'anhydride acétique.

8. Procédé de préparation de composés de formule générale I telle que définie à la revendication 1, dans laquelle W est O, $R_4$ est H et $R_5$ est

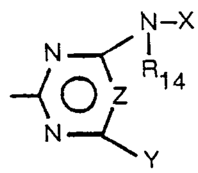

[où $R_{14}$ est H, Z et Y sont tels que définis à la revendication 1, et X est

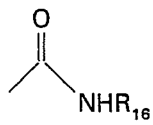

(où $R_{16}$ est tel que défini à la revendication 1)], qui consiste à faire réagir un composé de formule XIV tel que défini à la revendication 7 avec un composé de formule:

$$R_{16}—NCO$$

(où $R_{16}$ est tel que défini à la revendication 1).

9. Procédé de préparation de composés de formule générale I telle que définie à la revendication 1, dans laquelle W est O, $R_4$ est H et $R_5$ est

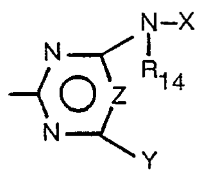

[où $R_{14}$ est H, Z et Y sont tels que définis à la revendication 1, et X est

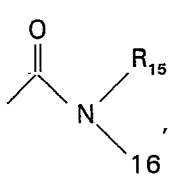

,

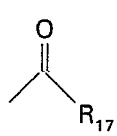

$CH_3SO_2$ ou $CF_3SO_2$ (où $R_{15}$, $R_{16}$ et $R_{17}$ sont tels que définis à la revendication 1)], qui consiste à faire réagir un composé de formule XIV tel que défini à la revendication 7, avec un composé de formule:

$$X—Cl$$

(dans laquelle X est tel que défini plus haut).

10. Composition propre à maîtriser la croissance de végétation indésirable, qui comprend un

composé de formule I tel que défini à la revendication 1, ainsi qu'au moins un agent tensio-actif et/ou un diluant solide ou liquide.

11. Procédé pour maîtriser la croissance de végétation indésirable en un lieu, qui consiste à appliquer à ce lieu une quantité efficace d'un composé de formule I tel que défini à la revendication 1.